# EUROPEAN PATENT APPLICATION

(11) **EP 4 299 757 A2**
(43) Date of publication of application: **03.01.2024**
(21) Application number: 23210217.8
(22) Date of filing: 12.11.2019
(51) Int. Cl.: C12Q 1/6806

(54) **DIRECTIONAL TARGETED SEQUENCING**

(30) Priority: 13.11.2018 US 201862760833 P
(62) Divisional of application: 19836181.8
(71) Applicant: Idbydna Inc., San Francisco CA 94107 (US)
(72) Inventor: MATSUZAKI, Hajime, California, 90275 (US); MEI, Yuying, Sunnyvale, 94087 (US); LIAO, Guochun, Belmont, 94002 (US)
(74) Representative: Mewburn Ellis LLP

(57) **Abstract**

The present disclosure provides methods and systems for processing nucleic acid molecules. The methods may comprise performing one or more extension or amplification processes to provide libraries for subsequent analysis using nucleic acid sequencing. Logical partitioning and directionality considerations may facilitate efficient and cost-effective amplification of target nucleic acid sequences.

## Description

### CROSS-REFERENCE

This application claims the benefit of U.S. Provisional Application No. 62/760,833, filed November 13, 2018, which application is incorporated herein by reference.

### BACKGROUND

Samples may be analyzed for various purposes, including detecting the presence or amount of a target such as a nucleic acid molecule in a sample. Analysis of a sample comprising one or more nucleic acid molecules may involve sequencing the nucleic acid molecules, or portions or derivatives thereof. Sequencing may facilitate identification of contaminants and/or species of potential interest within a sample. For example, sequencing may be used to identify a microorganism or pathogen within a sample.

Methods of targeted sequencing on Next-generation sequencing (NGS) platforms such as Illumina, have generally been based on hybridization capture or polymerase chain reaction (PCR). Hybrid capture approaches typically use biotinylated ribonucleic acid (RNA) or deoxyribonucleic acid (DNA) probes together with streptavidin coated magnetic beads to enrich regions of interest within a genome. However, hybridization times may be long and stringent washes may be technically challenging and not readily amenable to automation. On the other hand, PCR approaches directly amplify regions of interest and may require less time and technical expertise and be easier to automate for high-throughput applications. While NGS methods facilitate sequencing large numbers of sequences at the same time, methods of efficiently and effectively sequencing large numbers of different sequences of interest are lacking.

### SUMMARY

Recognized herein is a need to improve targeted and multi-plexed sequencing methods. The methods described herein may enable a broad range of genomics applications including metagenomics, cancer diagnostics, human variation (pharmacogenomics and ancestry), and agricultural and food testing. The high levels of multiplexing per sample, combined with running multiple samples in a single sequencing run, may also facilitate a variety of high throughput applications.

Polymerase chain reaction (PCR) may be multiplexed to simultaneously sequence many regions of interest. Due to the accumulation of unproductive primer-dimers and unexpected non-specific products, there may be practical limits to the number of primer pairs used for Multiplex PCR. Such multiplexing limits may be overcome by methods that are based on ligation of oligos to targeted regions or enzymatic digestion of primer-dimers following Multiplex PCR. In a ligation-based method, an extended primer and a second downstream primer that are both hybridized to the target may be ligated. Alternatively, a double-stranded probe with single-stranded overhangs on both ends may hybridize to fragmented target DNA and ligation may be used to generate circular DNA of the targeted regions. The sensitivity of ligation-based methods may be limited by low amounts of target regions in the original sample. In contrast, sensitivity may be much higher when running Multiplex PCR and then enzymatically digesting the accumulated primer-dimers. Some methods such as AmpliSeq (Thermo Fisher) may involve removing primer sequences from the PCR amplicons prior to sequencing. Multiplexing levels of 24,000-plex have been achieved by AmpliSeq.

Nested PCR is a variation of PCR that increases specificity while maintaining sensitivity. A second set of primers may anneal to regions within the amplicon generated by a first set of primers targeting a region of interest. Unexpected non-specific amplicons from the first set of primers are unlikely to be templates for the second nested pair of primers. Similarly, the use of a third primer in a hybridization-based amplicon detection method such as TaqMan (Thermo Applied) increases the specificity of quantitative PCR (qPCR). The term Asymmetric PCR refers to PCR using only one primer or very limited quantities of a second primer in a pair. Unlike PCR which results in geometric amplification of both amplicon strands, Asymmetric PCR results in linear yields of one amplicon strand.

Sequencing PCR amplicons using an NGS platform such as the Illumina NGS platform may require the addition of sequencing adapters. For example, flowcell attachment sequences may be added to the ends of amplicons that enable amplification (e.g., bridge amplification) to generate clonal clusters. Similarly, sequencing primers or complements thereof, such as those used in a sequencing-by-synthesis process, may also be added. These sequencing adapters (e.g., pairs of Illumina attachment sequences and complementary primer binding sites) may be part of PCR primer designs. Examples of such primers include those used in TruSeq panels and those added by ligation to amplicons such as in AmpliSeq protocols adapted for the Illumina platform.

The present disclosure provides methods of Directional Targeted Sequencing. The methods disclosed herein comprise Multiplex Biotinylated Asymmetric PCR. The methods may enable simultaneous sequencing of thousands of regions of interest corresponding to nucleic acid molecules from a nucleic acid sample. Sensitivity to detect low amounts of targets in a sample is driven by Multiplex PCR, while subsequent Asymmetric PCR provides increased specificity. Logical partitioning and directionality considerations may be used to facilitate these processes. The presently claimed methods may allow for high through put sequencing of various target sequences without requiring the use of ligation or enzymatic digestion methods.

The present disclosure provides methods and systems for analyzing nucleic acid samples comprising one or more nucleic acid molecules comprising one or more target nucleic acid sequences. The target nucleic acid sequences may correspond to one or more pathogens (e.g., bacteria, viruses, fungi, or parasites) and/or may be associated with one or more diseases or disorders. Methods of analyzing the nucleic acid molecules may involve one or more processes such as one or more extension or amplification reactions. For example, the methods described herein may involve one or more nucleic acid amplification reactions. Through various design considerations, the one or more target nucleic acid sequences of the one or more nucleic acid molecules may be analyzed with high sensitivity and specificity.

In an aspect, the present disclosure provides a method of analyzing a nucleic acid sample, comprising: (a) providing a sample comprising a plurality of nucleic acid molecules, which plurality of nucleic acid molecules comprise a target nucleic acid sequence; (b) subjecting said plurality of nucleic acid molecules to a condition sufficient to generate one or more copies of said target nucleic acid sequence, or complements thereof, wherein generating said one or more first copies, or complements thereof, comprises annealing first primers of a plurality of first primers and second primers of a plurality of second primers to nucleic acid molecules of said plurality of nucleic acid molecules; (c) separating said one or more first copies of said target nucleic acid sequence, or complements thereof, from other materials; (d) subjecting said one or more first copies of said target nucleic acid sequence, or complements thereof, to a condition sufficient to generate one or more second copies of said target nucleic acid sequence, or complements thereof, wherein generating said one or more second copies, or complements thereof, comprises annealing third primers of a plurality of third primers to said one or more first copies, or complements thereof; (e) separating said one or more second copies of said target nucleic acid sequence, or complements thereof, from other materials; (f) subjecting said one or more second copies of said target nucleic acid sequence, or complements thereof, to conditions sufficient to generate one or more third copies of said target nucleic acid sequence, or complements thereof, wherein generating said one or more third copies, or complements thereof, comprises annealing first sequencing adapters of a plurality of first sequencing adapters and second sequencing adapters of a plurality of second sequencing adapters to said one or more second copies, or complements thereof, wherein said plurality of third primers are configured to anneal to said one or more first copies, or complements thereof, downstream of said first primers or second primers, or complements thereof, wherein said plurality of third primers comprise a portion of a first sequencing adapter of said plurality of first sequencing adapters, or a complement thereof.

In some embodiments, (c) comprises using a purification column. In some embodiments, (e) comprises using a purification column.

In some embodiments, first primers of said plurality of first primers and/or second primers of said plurality of second primers comprise a biotin moiety. In some embodiments, first primers of said plurality of first primers comprise said biotin moiety. In some embodiments, first copies, or complements thereof, of said one or more first copies, or complements thereof, comprise said biotin moiety. In some embodiments, (c) comprises using streptavidin-coated beads. In some embodiments, said beads are magnetic beads. In some embodiments, (c) comprises bringing nucleic acid molecules into contact with said beads, thereby attaching nucleic acid molecules to said beads, and using a magnet to separate said nucleic acid molecules attached to said beads from nucleic acid molecules not attached to said beads.

In some embodiments, each first sequencing adapter of said plurality of first sequencing adapters and each second sequencing adapter of said plurality of second sequencing adapters comprise an index sequence. In some embodiments, said index sequence comprises a barcode sequence. In some embodiments, said index sequence comprises between 4 and 20 nucleotides. In some embodiments, index sequences of said plurality of first sequencing adapters and said plurality of second sequencing adapters are different from one another.

In some embodiments, each first sequencing adapter of said plurality of first sequencing adapters and each second sequencing adapter of said plurality of second sequencing adapters comprise a flowcell attachment sequence. In some embodiments, said flowcell attachment sequence of said plurality of first sequencing adapters is different than said flowcell attachment sequence of said plurality of second sequencing adapters.

In some embodiments, each first sequencing adapter of said plurality of first sequencing adapters comprises a sequencing primer, and wherein each third primer of said plurality of third primers comprises said sequencing primer, or a portion thereof.

In some embodiments, each first sequencing adapter of said plurality of first sequencing adapters and each second sequencing adapter of said plurality of second sequencing adapters comprise a sequencing primer. In some embodiments, said sequencing primer of said plurality of first sequencing adapters is different than said sequencing primer of said plurality of second sequencing adapters.

In some embodiments, in (b), said condition comprises two or more different temperatures. In some embodiments, (b) comprises thermal cycling.

In some embodiments, in (d), said condition comprises two or more different temperatures. In some embodiments, (d) comprises thermal cycling.

In some embodiments, in (f), said condition comprises two or more different temperatures. In some embodiments, (f) comprises thermal cycling.

In some embodiments, said sample comprises a bodily fluid. In some embodiments, said bodily fluid is selected from the group consisting of blood, urine, saliva, and sweat.

In some embodiments, said plurality of nucleic acid molecules of said sample comprises a plurality of deoxyribonucleic acid (DNA) molecules.

In some embodiments, said plurality of nucleic acid molecules of said sample comprises a plurality of ribonucleic acid (RNA) molecules.

In some embodiments, said sample comprises one or more cells. In some embodiments, the method further comprises lysing said one or more cells of said sample.

In some embodiments, said sample derives from a patient. In some embodiments, said patient has or is suspected of having a disease or disorder.

In some embodiments, said sample derives from a plurality of patients. In some embodiments, said plurality of patients has or are suspected of having a disease or disorder.

In some embodiments, said target nucleic acid sequence corresponds to a pathogen. In some embodiments, said pathogen is selected from the group consisting of a fungus, bacterium, virus, and parasite.

In another aspect, the present disclosure provides a method of analyzing a nucleic acid sample, comprising: (a) providing a sample comprising a plurality of nucleic acid molecules, which plurality of nucleic acid molecules comprise a target nucleic acid sequence; (b) subjecting said plurality of nucleic acid molecules to a condition sufficient to generate one or more copies of said target nucleic acid sequence, or complements thereof, wherein generating said one or more first copies, or complements thereof, comprises annealing first primers of a plurality of first primers and second primers of a plurality of second primers to nucleic acid molecules of said plurality of nucleic acid molecules; (c) separating said one or more first copies of said target nucleic acid sequence, or complements thereof, from other materials; and (d) subjecting said one or more first copies of said target nucleic acid sequence, or complements thereof, to a condition sufficient to generate one or more second copies of said target nucleic acid sequence, or complements thereof, wherein generating said one or more second copies, or complements thereof, comprises annealing third primers of a plurality of third primers to said one or more first copies, or complements thereof, wherein said plurality of third primers are configured to anneal to said one or more first copies, or complements thereof, downstream of said first primers or second primers, or complements thereof; wherein said plurality of third primers comprise a portion of a first sequencing adapter of a plurality of first sequencing adapters, or a complement thereof.

In some embodiments, the method further comprises separating said one or more second copies of said target nucleic acid sequence, or complements thereof, from other materials. In some embodiments, separating said one or more second copies of said target nucleic acid sequence, or complements thereof, comprises using a purification column.

In some embodiments, (c) comprises using a purification column.

In some embodiments, wherein first primers of said plurality of first primers and/or second primers of said plurality of second primers comprises a biotin moiety. In some embodiments, first primers of said plurality of first primers comprise said biotin moiety. In some embodiments, first copies, or complements thereof, of said one or more first copies, or complements thereof, comprise said biotin moiety. In some embodiments, (c) comprises using streptavidin-coated beads. In some embodiments, said beads are magnetic beads. In some embodiments, (c) comprises bringing nucleic acid molecules into contact with said beads, thereby attaching nucleic acid molecules to said beads, and using a magnet to separate said nucleic acid molecules attached to said beads from nucleic acid molecules not attached to said beads.

In some embodiments, each first sequencing adapter of said plurality of first sequencing adapters comprises an index sequence. In some embodiments, said index sequence comprises a barcode sequence. In some embodiments, said index sequence comprises between 4 and 20 nucleotides.

In some embodiments, each first sequencing adapter of said plurality of first sequencing adapters comprises a flowcell attachment sequence.

In some embodiments, each first sequencing adapter of said plurality of first sequencing adapters comprises a sequencing primer, and wherein each third primer of said plurality of third primers comprises said sequencing primer, or a portion thereof.

In some embodiments, in (b), said condition comprises two or more different temperatures. In some embodiments, (b) comprises thermal cycling.

In some embodiments, in (d), said condition comprises two or more different temperatures. In some embodiments, (b) comprises thermal cycling.

In some embodiments, said sample comprises a bodily fluid. In some embodiments, said bodily fluid is selected from the group consisting of blood, urine, saliva, and sweat.

In some embodiments, said plurality of nucleic acid molecules of said sample comprises a plurality of deoxyribonucleic acid (DNA) molecules.

In some embodiments, said plurality of nucleic acid molecules of said sample comprises a plurality of ribonucleic acid (RNA) molecules.

In some embodiments, said sample comprises one or more cells. In some embodiments, the method further comprises lysing said one or more cells of said sample.

In some embodiments, said sample derives from a patient. In some embodiments, said patient has or is suspected of having a disease or disorder.

In some embodiments, said sample derives from a plurality of patients. In some embodiments, said plurality of patients has or are suspected of having a disease or disorder.

In some embodiments, said target nucleic acid sequence corresponds to a pathogen. In some embodiments, said pathogen is selected from the group consisting of a fungus, bacterium, virus, and parasite.

In another aspect, the present disclosure provides a method of analyzing a nucleic acid sample, comprising:(a) providing a sample comprising a plurality of nucleic acid molecules, which plurality of nucleic acid molecules comprise a target nucleic acid sequence; (b) subjecting said plurality of nucleic acid molecules to a condition sufficient to generate one or more copies of said target nucleic acid sequence, or complements thereof, wherein generating said one or more first copies, or complements thereof, comprises annealing first primers of a plurality of first primers and second primers of a plurality of second primers to nucleic acid molecules of said plurality of nucleic acid molecules; (c) separating said one or more first copies of said target nucleic acid sequence, or complements thereof, from other materials; (d) subjecting said one or more first copies of said target nucleic acid sequence, or complements thereof, to conditions sufficient to generate one or more second copies of said target nucleic acid sequence, or complements thereof, wherein generating said one or more second copies, or complements thereof, comprises annealing first sequencing adapters of a plurality of first sequencing adapters and second sequencing adapters of a plurality of second sequencing adapters to said one or more first copies, or complements thereof, wherein said plurality of first primers comprise (i) a portion of a first sequencing adapter of said plurality of first sequencing adapters, or a complement thereof; or (ii) a filler sequence that does not have substantial sequence identity to a natural sequence.

In some embodiments, (c) comprises using a purification column.

In some embodiments, first primers of said plurality of first primers and/or second primers of said plurality of second primers comprise a biotin moiety. In some embodiments, first primers of said plurality of first primers comprise said biotin moiety. In some embodiments, first copies, or complements thereof, of said one or more first copies, or complements thereof, comprise said biotin moiety. In some embodiments, (c) comprises using streptavidin-coated beads. In some embodiments, said beads are magnetic beads. In some embodiments, (c) comprises bringing nucleic acid molecules into contact with said beads, thereby attaching nucleic acid molecules to said beads, and using a magnet to separate said nucleic acid molecules attached to said beads from nucleic acid molecules not attached to said beads.

In some embodiments, each first sequencing adapter of said plurality of first sequencing adapters and each second sequencing adapter of said plurality of second sequencing adapters comprise an index sequence. In some embodiments, said index sequence comprises a barcode sequence. In some embodiments, said index sequence comprises between 4 and 20 nucleotides. In some embodiments, index sequences of said plurality of first sequencing adapters and said plurality of second sequencing adapters are different from one another.

In some embodiments, each first sequencing adapter of said plurality of first sequencing adapters and each second sequencing adapter of said plurality of second sequencing adapters comprise a flowcell attachment sequence. In some embodiments, said flowcell attachment sequence of said plurality of first sequencing adapters is different than said flowcell attachment sequence of said plurality of second sequencing adapters.

In some embodiments, each first sequencing adapter of said plurality of first sequencing adapters comprises a sequencing primer, and wherein each first primer of said plurality of first primers comprises said sequencing primer, or a portion thereof.

In some embodiments, each first sequencing adapter of said plurality of first sequencing adapters and each second sequencing adapter of said plurality of second sequencing adapters comprise a sequencing primer. In some embodiments, said sequencing primer of said plurality of first sequencing adapters is different than said sequencing primer of said plurality of second sequencing adapters.

In some embodiments, each first primer of said plurality of first primers comprises said filler sequence. In some embodiments, said filler sequence has 25% or lower sequence identity to a natural sequence. In some embodiments, said filler sequence has 15% or lower sequence identity to a natural sequence. In some embodiments, said filler sequence has 10% or lower sequence identity to a natural sequence. In some embodiments, said filler sequence has 5% or lower sequence identity to a natural sequence.

In some embodiments, in (b), said condition comprises two or more different temperatures. In some embodiments, (b) comprises thermal cycling.

In some embodiments, in (d), said condition comprises two or more different temperatures. In some embodiments, (b) comprises thermal cycling.

In some embodiments, said sample comprises a bodily fluid. In some embodiments, said bodily fluid is selected from the group consisting of blood, urine, saliva, and sweat.

In some embodiments, said plurality of nucleic acid molecules of said sample comprises a plurality of deoxyribonucleic acid (DNA) molecules.

In some embodiments, said plurality of nucleic acid molecules of said sample comprises a plurality of ribonucleic acid (RNA) molecules.

In some embodiments, said sample comprises one or more cells. In some embodiments, the method further comprises lysing said one or more cells of said sample.

In some embodiments, said sample derives from a patient. In some embodiments, said patient has or is suspected of having a disease or disorder.

In some embodiments, said sample derives from a plurality of patients. In some embodiments, said plurality of patients have or are suspected of having a disease or disorder.

In some embodiments, said target nucleic acid sequence corresponds to a pathogen. In some embodiments, said pathogen is selected from the group consisting of a fungus, bacterium, virus, and parasite.

In a further aspect, the present disclosure provides a method of analyzing a nucleic acid sample, comprising: (a) providing a sample comprising a plurality of nucleic acid molecules, which plurality of nucleic acid molecules comprise a target nucleic acid sequence, or a complement thereof, wherein a subset of said plurality of nucleic acid molecules further comprise a directing sequence; (b) subjecting said plurality of nucleic acid molecules to a condition sufficient to generate one or more first copies of said target nucleic acid sequence, or complements thereof, wherein generating said one or more first copies, or complements thereof, comprises annealing first primers of a plurality of first primers to said plurality of nucleic acid molecules; (c) separating said one or more first copies of said target nucleic acid sequence, or complements thereof, from other materials; and (d) subjecting said one or more first copies of said target nucleic acid sequence, or complements thereof, to conditions sufficient to generate one or more second copies of said target nucleic acid sequence, or complements thereof, wherein generating said one or more second copies, or complements thereof, comprises annealing first sequencing adapters of a plurality of first sequencing adapters and second sequencing adapters of a plurality of second sequencing adapters to said one or more first copies, or complements thereof, wherein said plurality of first primers are configured to anneal to said plurality of nucleic acid molecules downstream of said directing sequence; wherein said plurality of first primers comprise a portion of a first sequencing adapter of said plurality of first sequencing adapters, or a complement thereof.

In some embodiments, the method further comprises prior to (a), generating said plurality of nucleic acid molecules comprising said target nucleic acid sequence, or a complement thereof, and said directing sequence.

In some embodiments, generating said plurality of nucleic acid molecules comprises performing one or more amplification reactions. In some embodiments, said one or more amplification reactions comprise one or more polymerase chain reactions.

In some embodiments, (c) comprises using a purification column.

In some embodiments, each first sequencing adapter of said plurality of first sequencing adapters and each second sequencing adapter of said plurality of second sequencing adapters comprise an index sequence. In some embodiments, said index sequence comprises a barcode sequence. In some embodiments, said index sequence comprises between 4 and 20 nucleotides. In some embodiments, index sequences of said plurality of first sequencing adapters and said plurality of second sequencing adapters are different from one another.

In some embodiments, each first sequencing adapter of said plurality of first sequencing adapters and each second sequencing adapter of said plurality of second sequencing adapters comprise a flowcell attachment sequence. In some embodiments, the flowcell attachment sequence of said plurality of first sequencing adapters is different than said flowcell attachment sequence of said plurality of second sequencing adapters.

In some embodiments, each first sequencing adapter of said plurality of first sequencing adapters comprises a sequencing primer, and wherein each first primer of said plurality of first primers comprises said sequencing primer, or a portion thereof.

In some embodiments, each first sequencing adapter of said plurality of first sequencing adapters and each second sequencing adapter of said plurality of second sequencing adapters comprise a sequencing primer. In some embodiments, said sequencing primer of said plurality of first sequencing adapters is different than said sequencing primer of said plurality of second sequencing adapters.

In some embodiments, in (b), said condition comprises two or more different temperatures. In some embodiments, (b) comprises thermal cycling.

In some embodiments, in (d), said condition comprises two or more different temperatures. In some embodiments, (b) comprises thermal cycling.

In some embodiments, said sample comprises a bodily fluid. In some embodiments, said bodily fluid is selected from the group consisting of blood, urine, saliva, and sweat.

In some embodiments, said plurality of nucleic acid molecules of said sample comprises a plurality of deoxyribonucleic acid (DNA) molecules.

In some embodiments, said plurality of nucleic acid molecules of said sample comprises a plurality of ribonucleic acid (RNA) molecules.

In some embodiments, said sample comprises one or more cells. In some embodiments, the method further comprises lysing said one or more cells of said sample.

In some embodiments, said sample derives from a patient.

In some embodiments, said patient has or is suspected of having a disease or disorder.

In some embodiments, said sample derives from a plurality of patients. In some embodiments, said plurality of patients has or are suspected of having a disease or disorder.

In some embodiments, said target nucleic acid sequence corresponds to a pathogen. In some embodiments, said pathogen is selected from the group consisting of a fungus, bacterium, virus, and parasite.

In another aspect, the present disclosure provides a method of analyzing a nucleic acid sample, comprising: (a) providing a sample comprising a plurality of nucleic acid molecules, which plurality of nucleic acid molecules comprise a target nucleic acid sequence; (b) subjecting said plurality of nucleic acid molecules to a condition sufficient to generate one or more first copies of said target nucleic acid sequence, or complements thereof, which one or more copies, or complements thereof, comprise a first primer sequence or complement thereof at a first end and a second primer sequence or complement thereof at a second end, wherein said first primer sequence comprises a biotin moiety; (c) separating said one or more first copies of said plurality of target nucleic acid sequences, or complements thereof, from other materials; (d) subjecting said one or more first copies of said plurality of target nucleic acid sequences, or complements thereof, to a condition sufficient to generate one or more second copies of said target nucleic acid sequence, or complements thereof, which one or more second copies, or complements thereof, comprise a third primer sequence or complement thereof at said first end and said first primer sequence or complement thereof at said first end; (e) separating said one or more second copies of said target nucleic acid sequence, or complements thereof, from other materials; and (f) subjecting said one or more second copies of said target nucleic acid sequence, or complements thereof, to a condition sufficient to generate one or more third copies of said target nucleic acid sequence, or complements thereof, which one or more third copies, or complements thereof, comprise a first sequencing adapter at a first end and a second sequencing adapter at a second end.

In some embodiments, (b) comprises annealing first primers of a plurality of first primers and second primers of a plurality of second primers to nucleic acid molecules of said plurality of nucleic acid molecules.

In some embodiments, (d) comprises annealing third primers of a plurality of third primers to said one or more first copies, or complements thereof.

In some embodiments, (f) comprises annealing first sequencing adapters of a plurality of first sequencing adapters and second sequencing adapters of a plurality of second sequencing adapters to said one or more second copies, or complements thereof.

In some embodiments, said plurality of third primers are configured to anneal to said one or more first copies, or complements thereof, downstream of said first primers or complements thereof.

In some embodiments, said plurality of third primers comprises a portion of a first sequencing adapter of said plurality of first sequencing adapters, or a complement thereof.

In some embodiments, (c) comprises using a purification column.

In some embodiments, (e) comprises using a purification column.

In some embodiments, (c) comprises using streptavidin-coated beads. In some embodiments, said beads are magnetic beads. In some embodiments, (c) comprises bringing nucleic acid molecules into contact with said beads, thereby attaching nucleic acid molecules to said beads, and using a magnet to separate said nucleic acid molecules attached to said beads from nucleic acid molecules not attached to said beads.

In some embodiments, said first sequencing adapter comprises a first index sequence and said second sequencing adapter comprises a second index sequence. In some embodiments, said first and second index sequences comprise barcode sequences. In some embodiments, said first and second index sequences comprise between 4 and 20 nucleotides. In some embodiments, said first index sequence is different from said second index sequence.

In some embodiments, said first sequencing adapter comprises a first flowcell attachment sequence and said second sequencing adapter comprises a second flowcell attachment sequence. In some embodiments, said first flowcell attachment sequence is different from said second flowcell attachment sequence.

In some embodiments, each first sequencing adapter of said plurality of first sequencing adapters comprises a sequencing primer, and wherein each third primer of said plurality of third primers comprises said sequencing primer, or a portion thereof.

In some embodiments, said first sequencing adapter comprises a first sequencing primer and said second sequencing adapter comprises a second sequencing primer. In some embodiments, said first sequencing primer is different from said second sequencing primer.

In some embodiments, in (b), said condition comprises two or more different temperatures. In some embodiments, (b) comprises thermal cycling.

In some embodiments, in (d), said condition comprises two or more different temperatures. In some embodiments, (b) comprises thermal cycling.

In some embodiments, in (f), said condition comprises two or more different temperatures. In some embodiments, (f) comprises thermal cycling.

In some embodiments, said sample comprises a bodily fluid. In some embodiments, said bodily fluid is selected from the group consisting of blood, urine, saliva, and sweat.

In some embodiments, said plurality of nucleic acid molecules of said sample comprises a plurality of deoxyribonucleic acid (DNA) molecules.

In some embodiments, said plurality of nucleic acid molecules of said sample comprises a plurality of ribonucleic acid (RNA) molecules.

In some embodiments, said sample comprises one or more cells. In some embodiments, the method further comprises lysing said one or more cells of said sample.

In some embodiments, said sample derives from a patient. In some embodiments, said patient has or is suspected of having a disease or disorder.

In some embodiments, said sample derives from a plurality of patients. In some embodiments, said plurality of patients has or are suspected of having a disease or disorder.

In some embodiments, said target nucleic acid sequence corresponds to a pathogen. In some embodiments, said pathogen is selected from the group consisting of a fungus, bacterium, virus, and parasite.

In a further aspect, the present disclosure provides a kit comprising: (a) a plurality of first primers, which plurality of first primers are configured to anneal to nucleic acid molecules comprising a target nucleic acid sequence; (b) a plurality of second primers, which plurality of second primers are configured to anneal to nucleic acid molecules comprising complements of said target nucleic acid sequence; and (c) a plurality of third primers, which plurality of third primers are configured to anneal to nucleic acid molecules adjacent to said target nucleic acid sequence or complements thereof.

In some embodiments, all or a portion of said plurality of first primers or said plurality of second primers comprise a biotin moiety. In some embodiments, all or a portion of said plurality of first primers comprise said biotin moiety. In some embodiments, said biotin moiety is a desthiobiotin moiety. In some embodiments, the method further comprises a plurality of streptavidin-functionalized magnetic beads.

In some embodiments, said plurality of first primers or said plurality of second primers comprise all or a portion of a sequencing adapter. In some embodiments, said plurality of first primers or said plurality of second primers comprise all or a portion of a sequencing primer. In some embodiments, said plurality of first primers comprise said sequencing primer.

In some embodiments, the method further comprises a plurality of first sequencing adapters and a plurality of second sequencing adapters. In some embodiments, each first sequencing adapter of said plurality of first sequencing adapters comprises a first flowcell attachment sequence. In some embodiments, each first sequencing adapter of said plurality of first sequencing adapters comprises a first sequencing primer. In some embodiments, each first sequencing adapter of said plurality of first sequencing adapters comprises a first index sequence. In some embodiments, each second sequencing adapter of said plurality of second sequencing adapters comprises a second flowcell attachment sequence. In some embodiments, each second sequencing adapter of said plurality of second sequencing adapters comprises a second sequencing primer. In some embodiments, each second sequencing adapter of said plurality of second sequencing adapters comprises a second index sequence.

In some embodiments, said target nucleic acid sequence corresponds to a pathogen. In some embodiments, said pathogen is selected from the group consisting of a parasite, virus, bacterium, and fungus.

In some embodiments, the method further comprises one or more reagents selected from the group consisting of polymerases, buffers, solvents, and deoxyribonucleotides molecules (dNTPs).

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "figure" and "FIG." herein), of which:
**FIG. 1** schematically illustrates an exemplary Multiplex Polymerase Chain Reaction (PCR) process;
**FIG. 2** schematically illustrates an exemplary Asymmetric PCR process;
**FIG. 3** schematically illustrates an exemplary Flowcell PCR process;
**FIG. 4** schematically illustrates off-target products and primer-dimers from an exemplary Multiplex PCR process;
**FIG. 5** schematically illustrates off-target products and primer-dimers from an exemplary Asymmetric PCR process;
**FIG. 6** schematically illustrates exemplary applications comprising the use of split-primer annealing sites;
**FIG. 7** schematically illustrates that off-target products and primer-dimers may not be sequenced using a sequencing platform;
**FIG. 8** schematically illustrates an exemplary process for analyzing ribonucleic acid (RNA) molecules;
**FIGs. 9A-9C** schematically illustrate an exemplary method comprising Multiplex PCR (**FIG. 9A**), Asymmetric PCR (**FIG. 9B**), and FC-PCR (**FIG. 9C**) processes;
**FIGs. 10A-10D** schematically illustrate an exemplary method comprising a Multiplex PCR process (**FIG. 10A**), first and second Asymmetric PCR processes (**FIGs. 10B** and **10C**), and an FC-PCR process (**FIG. 10D**);
**FIGs. 11A-11D** schematically illustrate an exemplary method comprising a Multiplex PCR process (**FIG. 11A**), first and second Asymmetric PCR processes (**FIGs. 11B** and **11C**), and an FC-PCR process (**FIG. 11D**);
**FIG. 12** shows exemplary spacers for inclusion in primer molecules;
**FIGs. 13A-13E** schematically illustrate an exemplary method comprising a Multiplex PCR process (**FIG. 13A**), an avidin-biotin purification process (**FIG. 13B**), first and second Asymmetric PCR processes (**FIGs. 13C** and **13D**), and an FC-PCR process (**FIG. 13E**);
**FIGs. 14A-14C** schematically illustrate an exemplary method comprising a Multiplex PCR process (**FIG. 14A**), an Asymmetric PCR process (**FIG. 14B**), and addition of a filler sequence (**FIG. 14C**); and
**FIG. 15** shows a computer system that is programmed or otherwise configured to implement methods of the present disclosure herein.

The patent or application file contains at least one drawing executed in color. Copies of this patent or patent application publication with color drawing(s) will be provided by the Office upon request and payment of the necessary fee.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

Where values are described as ranges, it will be understood that such disclosure includes the disclosure of all possible sub-ranges within such ranges, as well as specific numerical values that fall within such ranges irrespective of whether a specific numerical value or specific sub-range is expressly stated.

The present disclosure provides methods of facilitating multiplexed sequencing of multiple regions of interest. The methods described herein may enable simultaneous sequencing of thousands of regions of interest. Multiple different amplification processes may be used to selectively amplify a plurality of target sequences of interest and generate a plurality of copies of the target sequences of interest, or complements thereof, comprising sequencing adapters. The plurality of copies, or complements thereof, may then be subjected to a nucleic acid sequencing process and the resultant sequencing reads used to detect the plurality of target sequences of interest. The plurality of target sequences of interest may comprise a plurality of different target sequences and may be sequenced at or near the same time.

The multiple different amplification processes used in the methods described herein may comprise, for example, Multiplex Polymerase Chain Reaction (PCR), Asymmetric PCR, and processing PCR such as Flowcell PCR (FC-PCR). One or more purification or separation processes may be used between different amplification processes to facilitate the preparation of nucleic acid molecules for sequencing. A Multiplex PCR process may be used to generate first copies, or complements thereof, of target sequences of interest from an initial plurality of nucleic acid molecules (e.g., deoxyribonucleic acid [DNA] or ribonucleic acid [RNA] molecules). These first copies, or complements thereof, may be purified to remove materials other than these first copies, or complements thereof. The purification process may be facilitated by the design of the primers used in the generation of the first copies, or complements thereof. The first copies, or complements thereof, of the target sequences of interest may then be subjected to an Asymmetric PCR process in which a single primer type is used to generate one or more second copies of the target sequences of interest, or complements thereof. The asymmetric primer may be configured to anneal to a first copy, or complement thereof, downstream of another primer sequence, or complement thereof (e.g., in a nested PCR process). The one or more second copies, or complements thereof, of the target sequences of interest may be subjected to a further purification process to remove materials other than these second copies, or complements thereof. The recovered second copies, or complements thereof, may then be subjected to an additional PCR process such as an FC-PCR process to generate one or more third copies, or complements thereof, including sequencing adapters. The sequencing adapters may including sequencing primers, index sequences (e.g., barcode sequences), and/or flowcell attachment sequences. The one or more third copies, or complements thereof, may then be subjected to a sequencing process and the resultant sequencing reads be used to detect the plurality of target sequences.

A method of analyzing a nucleic acid sample may comprise providing a sample comprising a plurality of nucleic acid molecules, which plurality of nucleic acid molecules comprise a plurality of target nucleic acid sequences. The plurality of nucleic acid molecules may then be subjected to conditions sufficient to generate one or more copies of the plurality of target nucleic acid sequences, or complements thereof. This process may comprise annealing first primers of a plurality of first primers and second primers of a plurality of second primers to nucleic acid molecules of the plurality of nucleic acid molecules. The one or more first copies of the plurality of target nucleic acid sequences, or complements thereof, may be separated from other materials such as off-target sequences and primer dimers using, for example, a purification column or biotin-avidin scheme. Such a biotin-avidin scheme may involve using biotin-tagged primers during the generation process and bringing the resultant biotin-tagged one or more first copies, or complements thereof, with streptavidin-functionalized magnetic beads. Magnetic separation may then be used to separate biotin-tagged materials from other materials.

The one or more first copies of the plurality of target nucleic acid sequences, or complements thereof, may then be subjected to conditions sufficient to generate one or more second copies of the plurality of target nucleic acid sequences, or complements thereof. This process may comprise annealing third primers of a plurality of third primers to the one or more first copies, or complements thereof. The plurality of third primers may be configured to anneal to the one or more first copies, or complements thereof, downstream of the first primers or second primers, or complements thereof. The plurality of third primers comprises a portion of a first sequencing adapter of the plurality of first sequencing adapters, or a complement thereof. The one or more second copies of the plurality of target nucleic acid sequences, or complements thereof, may then be separated from other materials including off-target sequences and primer dimers (e.g., using a purification column). The one or more second copies of the plurality of target nucleic acid sequences, or complements thereof, may be subjected to conditions sufficient to generate one or more third copies of the plurality of target nucleic acid sequences, or complements thereof, wherein generating the one or more third copies, or complements thereof, comprises annealing first sequencing adapters of a plurality of first sequencing adapters and second sequencing adapters of a plurality of second sequencing adapters to the one or more second copies, or complements thereof.

A method of analyzing a nucleic acid sample may comprise providing a sample comprising a plurality of nucleic acid molecules, which plurality of nucleic acid molecules comprise a plurality of target nucleic acid sequences. The plurality of nucleic acid molecules may then be subjected to conditions sufficient to generate one or more copies of the plurality of target nucleic acid sequences, or complements thereof. This process may comprise annealing first primers of a plurality of first primers and second primers of a plurality of second primers to nucleic acid molecules of the plurality of nucleic acid molecules. The one or more copies, or complements thereof, may comprise a first primer sequence or complement thereof at a first end and a second primer sequence or complement thereof at a second end, wherein the first primer sequence comprises a biotin moiety. The one or more first copies of the plurality of target nucleic acid sequences, or complements thereof, may be separated from other materials such as off-target sequences and primer dimers using, for example, a purification column or biotin-avidin scheme. Such a biotin-avidin scheme may involve using biotin-tagged primers during the generation process and brining the resultant biotin-tagged one or more first copies, or complements thereof, with streptavidin-functionalized magnetic beads. Magnetic separation may then be used to separate biotin-tagged materials from other materials.

The one or more first copies of the plurality of target nucleic acid sequences, or complements thereof, may then be subjected to conditions sufficient to generate one or more second copies of the plurality of target nucleic acid sequences, or complements thereof, which one or more second copies, or complements thereof, comprise a third primer sequence or complement thereof at the first end and the first primer sequence or complement thereof at the first end. This process may comprise annealing third primers of a plurality of third primers to the one or more first copies, or complements thereof. The plurality of third primers may comprise a portion of a first sequencing adapter, or a complement thereof. The one or more second copies of the plurality of target nucleic acid sequences, or complements thereof, may then be separated from other materials including off-target sequences and primer dimers (e.g., using a purification column). The one or more second copies of the plurality of target nucleic acid sequences, or complements thereof, may be subjected to conditions sufficient to generate one or more third copies of the plurality of target nucleic acid sequences, or complements thereof, which one or more third copies, or complements thereof, comprise a first sequencing adapter at a first end and a second sequencing adapter at a second end. This process may comprise annealing first sequencing adapters of a plurality of first sequencing adapters and second sequencing adapters of a plurality of second sequencing adapters to the one or more second copies, or complements thereof.

### Directional Targeted Sequencing

The methods described herein may involve one or more different amplification processes. For example, the methods may involve one or more different PCR processes. A PCR process may comprise the use of various reagents including, but not limited to, primers, probes, dNTPs, polymerases, solvents, and buffers. A PCR process may involve annealing a primer (e.g., a specific or non-specific primer) to a nucleic acid molecule and extending the primer using a polymerase to generate a complement of a sequence of the nucleic acid molecule. This process may be repeated one or more times. For example, a nucleic acid sample may be subjected to 1, 2, 3, 4, 5, 6, 7, 8, 9, 10, 12, 15, 18, 20, 25, 30, or more annealing and extension processes. Each annealing and extension process may occur at a given temperature or temperatures. A denaturing process may be used to separate a nucleic acid molecule from an extension product and to regenerate the nucleic acid molecule for additional annealing and extension processes. Denaturation of nucleic acid molecules may be brought about by a chemical, thermal, or other stimulus. A thermal denaturation process may occur at a different temperature than an annealing and/or extension process. Accordingly, a PCR process may comprise a plurality of annealing, extension, and denaturing processes and, consequently, thermal cycling between two or more different temperatures.

The one or more different PCR processes used in a directional targeted sequencing method may include, for example, Multiplex PCR, Asymmetric PCR, and Flowcell PCR (FC-PCR) processes. A directional targeted sequencing method may comprise performing a Multiplex PCR process followed by one or more Asymmetric PCR processes. An FC-PCR process may then be used to prepare resultant amplicons for analysis using nucleic acid sequencing. A plurality of nucleic acid molecules (e.g., DNA or RNA molecules) comprising a plurality of target nucleic acid sequences may be brought into contact with a first set of primers configured to anneal to a region in proximity to a target nucleic acid sequence or collection of target nucleic acid sequences and a second set of primers configured to anneal to a region in proximity to the complement of the target nucleic acid sequence or collection of target nucleic acid sequences. The first or second set of primers may comprise all or a portion of a sequencing adapter (such as a sequencing primer) useful for a sequencing. The sequencing adapter or portion thereof may be designed for use with a particular sequencing platform or may be generic (e.g., applicable for use with one or more different sequencing platforms). The plurality of nucleic acid molecules and the first and second set of primers may be subjected to conditions sufficient to anneal the first and second primers to nucleic acid molecules of the plurality of nucleic acid molecules. First and second primers may anneal to both nucleic acid molecules including one or more target nucleic acid sequences, or complements thereof, as well as nucleic acid molecules that do not comprise a target nucleic acid sequence, or complement thereof. Annealed first and second primers may be extended (e.g., using a polymerase and deoxyribonucleotides triphosphate molecules (dNTPs)) to generate extension products. The extension products hybridized to nucleic acid molecules of the plurality of nucleic acid molecules may be subjected to conditions sufficient to separate the extension products from the nucleic acid molecules (e.g., to denature the resultant double-stranded nucleic acid molecules). Additional rounds of annealing, extension, and denaturing may take place to generate a plurality of amplicons comprising target nucleic acid sequences or complements thereof. The number of cycles performed may be as few as one or greater than 25 cycles. For example, 1, 2, 3, 4, 5, 6, 7, 8, 9, 10, 15, 20, 25, or more cycles may be used in a Multiplex PCR process. In some cases, the first set of primers may comprise a biotin moiety. In other cases, the first set of primers may not include a biotin moiety but may be replaced or supplemented during later rounds of amplification with first primers that do comprise a biotin moiety. During the Multiplex PCR process, off-target amplicons and primer dimers may also be produced.

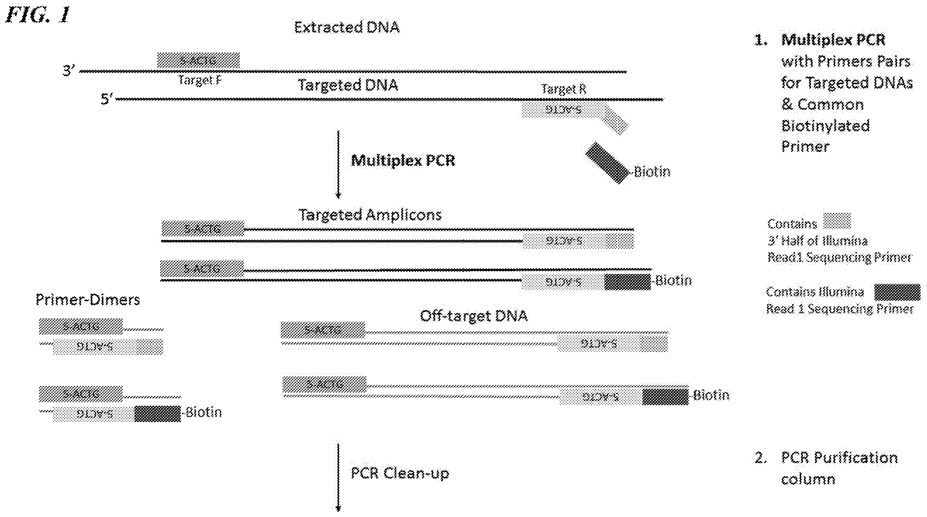

The Multiplex PCR process is illustrated in **FIG. 1****.** A pair of example targeting primers is shown in two shades of gray. For each locus of interest, the forward primer (dark gray) is unmodified, while the reverse primer (light gray) has the 3' half of the Illumina Read1 Sequencing Primer (light red). In the early cycles of PCR, these targeting primers drive the amplification. In later rounds, the common biotinylated primer (red) comes into play and adds a 5' Biotin to the reverse strand of the amplicons. This primer is referred to as "common" because it will anneal to the Illumina Sequencing Primer portion that is common to the 3' ends of the forward strands of all multiplex amplicons. Expected amplicons are shown with black lines, while unwanted primer-dimers and non-specific (off target) products are shown with green lines. The number of PCR cycles depends on the application but is typically 25 or more. At the completion of the Multiplex PCR cycles, the reaction is passed through a PCR purification column such as Qiagen QiaQuick. The PCR purification process may remove unused primers and primer-dimers formed during Multiplex PCR. The PCR amplicons may be eluted from the purification column in water or a buffered solution.

A purification process may be used to separate targeted amplicons (e.g., amplification/extension products comprising a target nucleic acid sequence or complement thereof) from other materials. A purification column may be used. Alternatively or in addition, an avidin-biotin scheme may be used to capture amplicons comprising a biotin moiety. Avidin-functionalized (e.g., streptavidin functionalized) beads may be brought into contact with the materials from the Multiplex PCR process such that biotin moieties attach to the avidin moieties of the beads. The beads may then be separated from other materials using, for example, a filtration or, in the case of magnetic beads, a magnetic separation process. Amplicons that do not comprise a biotin moiety may thus be separated and discarded. An Asymmetric PCR process may then be performed using a third set of primers. The third set of primers may be nested primers configured to anneal to a target amplicon downstream of a primer of the first or second sets of primers, or a complement thereof. The third set of primers may comprise a functional group or moiety such as all or a portion of a sequencing adapter, or complement thereof (e.g., a sequencing primer, or complement thereof). Upon annealing to a nucleic acid molecule, a third primer may be extended (e.g., using a polymerase and dNTPs) to generate a double-stranded nucleic acid molecule that may be subsequently subjected to conditions sufficient to denature it to generate the nucleic acid molecule and an extension product. Additional rounds of annealing, extension, and denaturing may take place to generate a plurality of amplicons comprising target nucleic acid sequences or complements thereof. The number of cycles performed may be as few as one or greater than 25 cycles. For example, 1, 2, 3, 4, 5, 6, 7, 8, 9, 10, 15, 20, 25, or more cycles may be used in a Asymmetric PCR process. An Asymmetric PCR process may comprise 15 cycles. The Asymmetric PCR process may be carried out on avidin-functionalized beads. An additional purification process such as a purification column method may be used to separate resultant nested target amplicons from other materials.

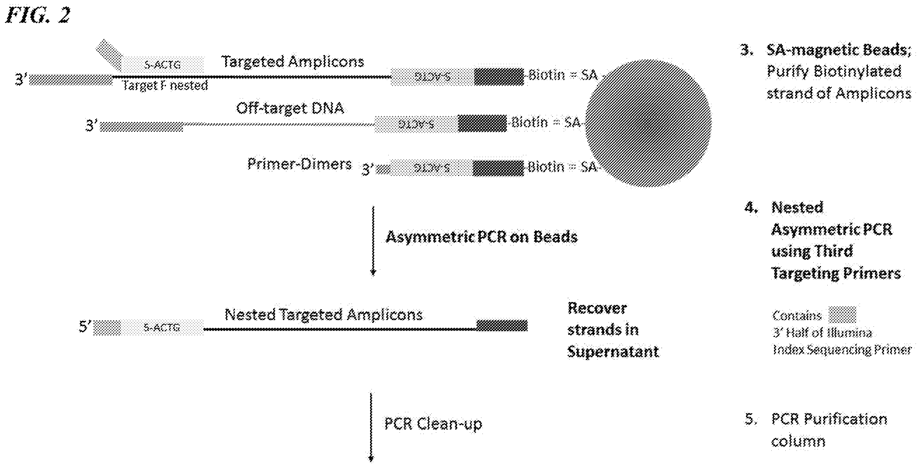

Biotin-based purification and Asymmetric PCR processes are illustrated in **FIG. 2****.** PCR amplicons are incubated with SA-magnetic beads and the biotinylated reverse strands of amplicons bind to the SA-beads. The biotinylated strands of remaining primer-dimers may also bind to the SA-beads. Upon pulling down the SA-beads with a magnet, all non-biotinylated products in solution may be discarded, including remaining non-biotinylated primer-dimers. Forward strands of amplicons may be hybridized to the bound biotinylated strands, but in the subsequent Asymmetric PCR step they will be denatured off. Because these forward strands do not contain any of the Illumina sequences, regardless of whether they are expected or unexpected non-specific products, these non-biotinylated strands will not be sequenced.

For each locus of interest, the third targeting primer (very light gray) has the 3' half of the Illumina Index Sequencing Primer (light blue). The third primer is designed to anneal downstream of the site of the Multiplex primer (5' portion of amplicon shown in dark gray). Over the cycles of Asymmetric PCR on the SA-beads, the third primer is extended to include the other Illumina sequence of the pair (3' portion shown in red). The number of cycles may depend on the application but may be no more than 15. The expected Nested Targeted amplicons may accumulate in solution off the beads. The supernatant may be recovered by pulling down the SA-beads. The supernatant may then be passed through a PCR purification column. The PCR purification process may remove unused third primers and primer-dimers formed during Asymmetric PCR. Since there may only be 15 cycles or less, the level of these primer-dimers may be low. Even if primer-dimers remain, they will not be sequenced because both ends have the same Illumina sequence and will not enable bridge amplification on the flowcell.

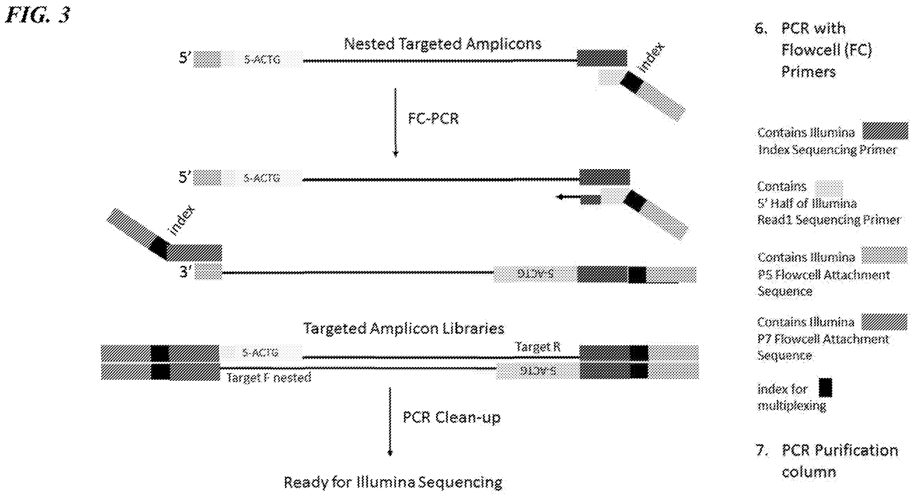

Nested target amplicons generated in an Asymmetric PCR process may comprise a first moiety at a first end comprising all or a portion of a first sequencing adapter, or complement thereof, and a second moiety at a second end comprising all or a portion of a second sequencing adapter, or complement thereof. For example, nested target amplicons may comprise a first sequencing primer, or complement thereof, at a first end and a second sequencing primer, or complement thereof, at a second end. The nested target amplicons may be subjected to an FC-PCR process to generate a plurality of target amplicons comprising first and second sequencing adapters (**FIG. 3**). The first and second sequencing adapters may comprise flowcell attachment sequences, index sequences, and sequencing primers. Flowcell attachment sequences of first and second sequencing adapters may be different from one another (e.g., P5 and P7 sequences of Illumina sequencing platforms). Similarly, index sequences and sequencing primers of first and second sequencing adapters may be the same or different from one another.

As shown in **FIG. 3****,** a primer with the 5' half of the Illumina Read1 Sequencing Primer (lighter red), index (black) and P5 Attachment sequence (yellow) may anneal to the 3' portion (red) of the forward strand of the Nested Targeted amplicons. This primer may be extended to generate the reverse strand of the amplicons. A primer with the full Illumina Index Sequencing Primer (blue), index (black), and P7 Attachment sequence (green) may anneal to the 5' portion (light blue) of the Nested Targeted amplicons. Index refers to short sequences that may be read during the Illumina SBS cycles and may allow assignment of reads to one sample library among a pool of many libraries. Additional details of index sequences are described elsewhere herein. After the FC-PCR, a PCR purification column may remove unused primers to provide amplicon libraries ready for sequencing.

An FC-PCR process may result in a plurality of target amplicons comprising a plurality of target nucleic acid sequences, or complements thereof. The resultant target amplicon library may then be subjected to a sequencing process to analyze the target sequences therein. The sequencing process may comprise, for example, sequencing by synthesis, sequencing by hybridization, sequencing by ligation, nanopore sequencing, or any other useful sequencing method. Any useful sequencing platform may be used, including, for example, an Illumina NGS platform.

### Library Preparation Scheme

The presently claimed methods may enable specificity, sensitivity, and high throughput analyses of nucleic acid samples. A plurality of logical partitioning and directionality considerations facilitate these desirable characteristics. Multiple mechanisms may facilitate such logical partitioning. For example, three targeting primers, including a pair for a Multiplex PCR process and a single primer for an Asymmetric PCR process may be used for each locus of interest. The Asymmetric PCR primer may be a nesting primer such that the annealing site is downstream of the annealing site of a primer used in the Multiplex PCR process. Accordingly, the Multiplex PCR primer should not be present in on-target amplicons. Similarly, the Asymmetric PCR primer should not anneal to non-specific/off target products. Unwanted products (e.g., off-target sequences, unamplified sequences, and/or primer dimers) may be physically separated from Multiplex amplicons (e.g., nucleic acid molecules generated in the Multiplex PCR process that comprise target nucleic acid sequences or complements thereof) to facilitate focused and efficient amplification of the target nucleic acid sequences. This may be achieved through, for example, biotinylating a strand of a multiplex amplicon and using streptavidin (SA)-coated magnetic beads to purify the biotinylated strand. Non-biotinylated strands may be discarded, regardless of whether the strands are from targeted amplicons or non-specific/off-target products. The Asymmetric PCR process may proceed on the SA-coated beads such that biotinylated Multiplex amplicons remain attached to the beads throughout the Asymmetric PCR process while Asymmetric amplicons (e.g., nucleic acid molecules generated in the Asymmetric PCR process that comprise target nucleic acid sequences or complements thereof) that are generated are not attached to the beads. A supernatant comprising the beads may be recovered by pulling down the SA-coated beads (e.g., using a magnet or magnetic component). Upon recovering the supernatant, the biotinylated strands bound to the SA-coated beads are discarded, regardless of whether the strands comprise target nucleic acid sequences or not. Finally, portions of the sequencing adapters used to construct amplicon libraries for sequencing on a sequencing platform (e.g., the Illumina platform) may be separately added to the ends of the Multiplex and Asymmetric amplicons. For example, a portion of a first sequencing adapter, or a complement thereof, may be incorporated into primers used in the Multiplex PCR process, and a portion of a second sequencing adapter, or complement thereof, may be incorporated into primers used in the Asymmetric PCR process. A final PCR process (e.g., FC-PCR) may then be used to add sequencing adapters to both ends of the Asymmetric amplicons. The sequencing adapters may comprise sequencing primers, flowcell attachment sequences, and index sequences (e.g., barcode sequences). After the final PCR process, the resultant amplicon libraries are ready to be pooled with amplicon libraries corresponding to other samples and sequenced on an Illumina platform sequencer, such as the MiSeq, NextSeq, or NovaSeq.

Directionality may also be facilitated by multiple mechanisms. For example, by biotinylating only one strand of the Multiplex PCR amplicons, directionality is established by temporarily giving preference to one strand over the other (e.g., a forward strand over a reverse strand). At this point, only the preferred biotinylated strand has Illumina sequence at one of the ends. Similarly, an Asymmetric PCR process carried out on the SA-coated beads only occurs in one direction of the amplicons. The Asymmetric primer (e.g., third targeting primer) may contain a portion of a sequencing adapter, or complement thereof, required pair for library construction. This component will only be added in the direction of the Asymmetric PCR primer extension such that newly created non-biotinylated strands will include a portion of a first sequencing adapter at a first end (added in the Multiplex PCR process) and a portion of a second sequencing adapter at a second end (added in the Asymmetric PCR process). These strands may now be given preference upon recovery of the supernatant in which they are included.

This Directionality mitigates the unwanted contributions of non-specific products from the Multiplex and Asymmetric PCR processes. Because of this Directionality, only a portion of one member of the pair of the sequencing adapters, or a complement thereof, may be added to these unexpected products. By lacking the full pair of sequencing adapters, these unexpected products will not support clonal amplification (e.g., bridge amplification, wildfire amplification, recombinase polymerase amplification, or other clonal amplification method) in a sequencing platform (e.g., an Illumina flowcell) and will not be sequenced. In contrast, Nested Asymmetric PCR amplicons comprising target nucleic acid sequences, or complements thereof, will comprise appropriate sequencing adapters and will be sequenced.

Unwanted contributions of non-specific products may be further mitigated by the use of split primer annealing sites corresponding to the portions of the sequencing adapters added to the ends of amplicons. Split primer annealing refers to primer designs that split the sequences of the sequencing adapters into two halves. Using a portion of each sequencing adapter (e.g., the 3' halves) or complement thereof in the Multiplex and Asymmetric PCR processes versus using another portion of the sequencing adapter or the full sequencing adapter (e.g., the 5' halves or full sequences) in the subsequent PCR process (e.g., FC-PCR process) may reduce the likelihood of sequencing residual unexpected non-specific products. Residual refers to products that were not completely washed away in a separation or purification process following the Multiplex PCR process (e.g., the SA-coated bead purification process) and were thus present during the Asymmetric PCR process.

The logical partitioning, directionality, and split annealing sites considerations described above may mitigate potentially problematic contributions from unwanted primer-dimers and non-specific or off-target products.

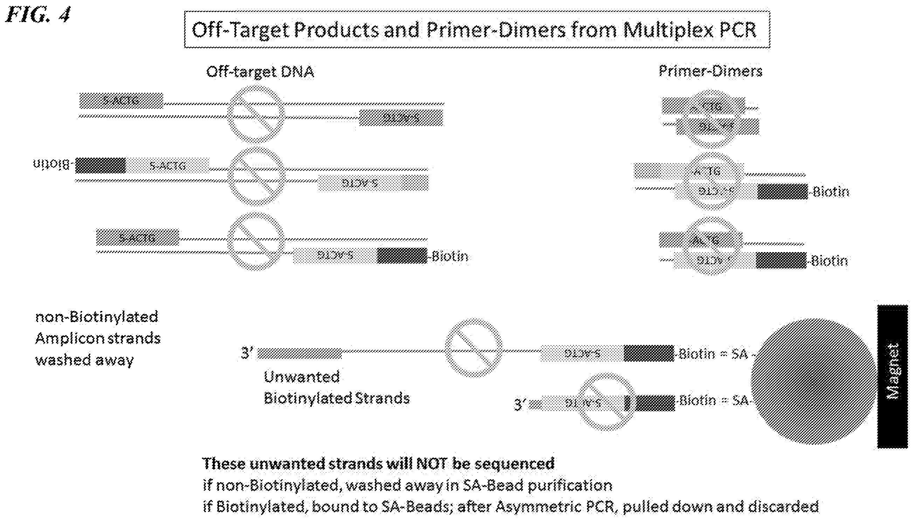

As shown in **FIG. 4****,** there may be numerous combinations of off-target products and primer-dimers from a Multiplex PCR process. Off-target multiplex amplicons may contain both non-biotinylated strands, one biotinylated strand and one non-biotinylated strand, or both biotinylated strands. Off-target strands may contain a complete sequencing primer (e.g., the full Illumina sequencing primer) (red), the 3' half of a sequencing primer (e.g., an Illumina sequencing primer) (light red), or sequencing primer or portion thereof. Regardless of the biotin or sequencing primer status of these off-target amplicon strands, none may be sequenced. This is because non-biotinylated strands may be washed away upon SA-Bead purification of the biotinylated strands; and, after an Asymmetric PCR process, biotinylated strands may be discarded upon recovery of the supernatant.

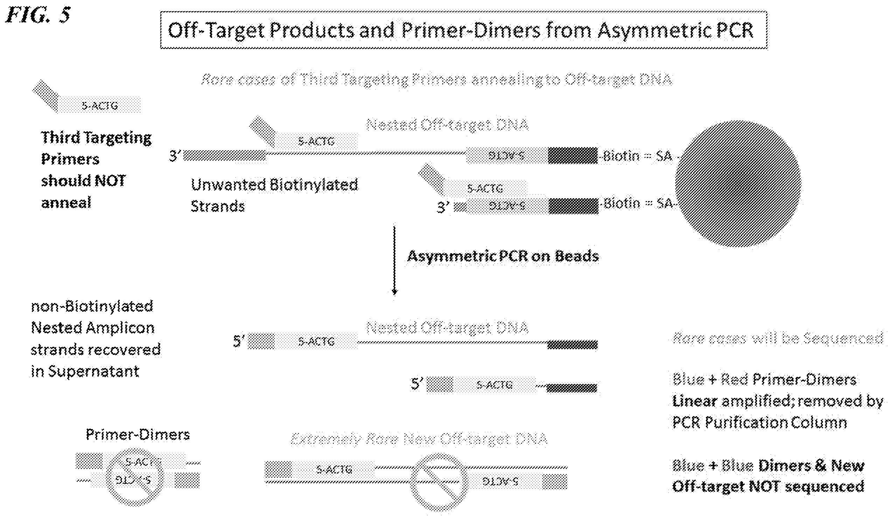

As shown in **FIG. 5****,** there may be cases where a third targeting primer may anneal to a non-specific biotinylated strand or dimer and be extended in the Asymmetric PCR process. This may generate amplicon strands shown with light blue on one end and red on the other end. These rare cases of an extended third primer will be sequenced. The extended biotinylated dimers (blue + red) are less likely to be sequenced because the Asymmetric PCR process is a linear amplification process and only yields limited amounts of amplicons which should be removed by a PCR Purification column. Primer-dimers may form from the third primers; these are shown with light blue on both ends. In extremely rare cases, longer non-specific amplicons may be generated when two different third primers amplify off a SA-bead bound strand. Because the Asymmetric PCR process may have no more than 15 cycles, the yields from geometric amplification will be limited. Even if present at higher amounts, these rare amplicons with light blue on both ends will not be sequenced, as described below.

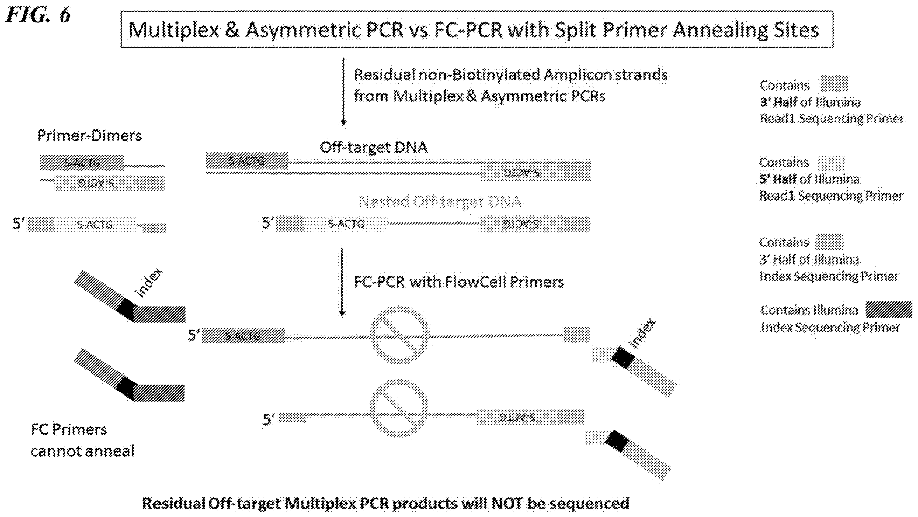

As shown in **FIG. 6****,** sequencing primers (e.g., the sequences corresponding to the Illumina SBS priming sites) may be split into two halves. The halves of the Read 1 priming site are shown as light red (3' half) and lighter red (5' half); and the Index priming site is shown in blue and the light blue (3' half). The residual non-biotinylated strands from the Multiplex PCR process will either have the 3' half of the Read1 site (light red) or no Illumina sequence. Residual means that these strands were not completely washed away upon SA-bead purification. In the Asymmetric PCR process, there is a small possibility that some of the third targeting primers annealed to and extended off these residual strands. This would create strands having the Index priming site (light blue) on one end and the Read1 priming site (light red) on the other end. But, since the FC-PCR primer only has the 5' half of the Read1 priming site (lighter red), the primer is unable to anneal to these blue + red strands. Since neither FC-PCR primer can anneal, these strands are not sequenced.

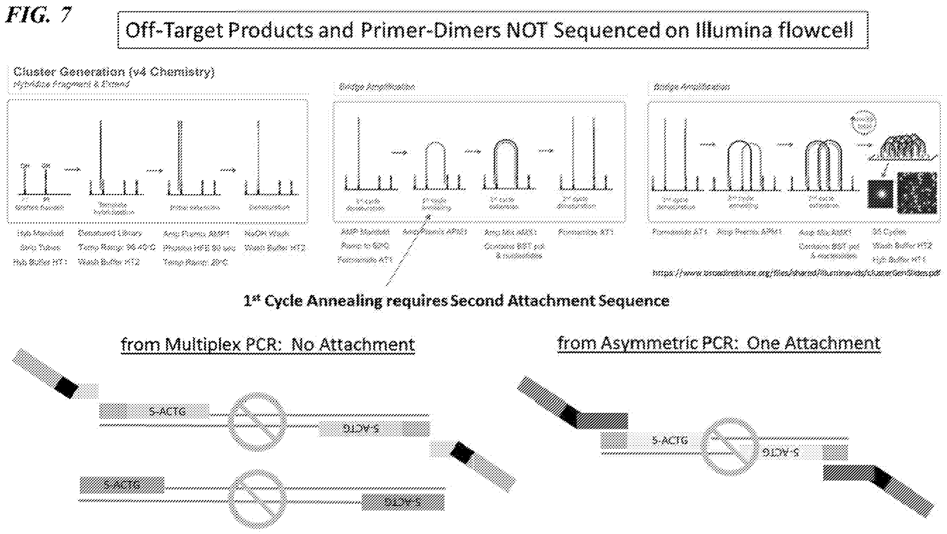

As shown in **FIG. 7****,** off-target primers and primer-dimers may not be sequenced. The upper panels depict the process of clonal cluster generation by bridge amplification employed using Illumina NGS platforms. Following the initial extension and denaturation of one strand of the amplicon library off the P7 attachment oligos grafted on the flowcell surface, the critical first cycle annealing step requires the extended amplicon strand to have sequences that are complementary to the P5 attachment oligos grafted on the flowcell surface. Off-target amplicon strands from the Multiplex PCR process may not have any of the attachment sequences because the FC-PCR primers may be unable to anneal. Off-target amplicon strands from the Asymmetric PCR process may only have one attachment sequence and therefore not enable bridge amplification on the flowcell surface. Some of these strands may contain the same attachment sequence (green) on both ends; however, bridge amplification will not occur because the second attachment sequence in the extended amplicon will be same orientation as the oligo grafted on the surface and cannot anneal.

### Samples

The presently claimed methods may involve analyzing one or more samples in sequence or in parallel. For example, a plurality of samples derived from a plurality of different sources may be analyzed in tandem, or a plurality of samples derived from a plurality of different sources may be separately analyzed. Alternatively, a plurality of samples derived from a single source (such as a single subject or patient) may be analyzed, e.g., at different time points. For example, a first sample may be derived from the source at a first time and a second sample may be derived from the source at a second time. Different time points may correspond to different phases of a treatment regimen, and/or before and after recovery or remission from a disease or disorder such as a cancer.

A nucleic acid sample may be an environmental sample, such as a sample collected from a surface, water source, or other feature. An environmental sample may have been handled or otherwise interacted with by a human or animal, and/or may include or be suspected of including a pathogen. Alternatively or in addition, a nucleic acid sample may derive from a patient (e.g., a human patient) or a plurality of patients. A patient from which a sample derives may have or be suspected of having a disease or disorder. In some cases, a patient from which a sample derives may have or be suspected of having a disease or disorder associated with a pathogen (e.g., bacteria, fungi, or virus). In some cases, a patient from which a sample derives may have been exposed or be suspected of having been exposed to a pathogen.

A sample may comprise or derive from a bodily fluid of an organism (e.g., human or animal) such as blood (e.g., whole blood, red blood cells, leukocytes or white blood cells, platelets), plasma, serum, sweat, tears, saliva, sputum, urine, mucus, semen, synovial fluid, breast milk, colostrum, amniotic fluid, bile, interstitial or extracellular fluid, bone marrow, or cerebrospinal fluid. For example, a sample may comprise or derive from a bodily fluid selected from the group consisting of blood, urine, saliva, and sweat. A sample may comprise or derive from a tissue such as an organ. A sample may be obtained from a subject (e.g., a human or animal) via intravenous or intraarterial methods, secretion collection, surgical extraction, swabbing, or any other method. A sample may comprise one or more cells comprising nucleic acid molecules, and/or may comprise cell-free nucleic acid molecules. Cells of a sample may be lysed to provide access to a plurality of nucleic acid molecules therein. A sample may undergo additional processing methods such as filtration, centrifugation, selective precipitation, and agitation prior to analysis of nucleic acid molecules included therein.

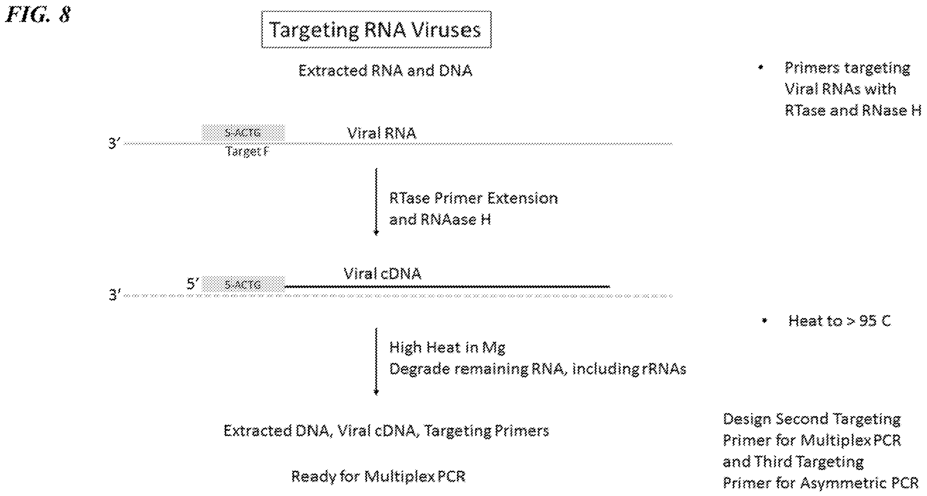

A sample may comprise a plurality of nucleic acid molecules. A nucleic acid molecule may be a single-stranded or a double-stranded nucleic acid molecule. Alternatively, a nucleic acid molecule may comprise both single-stranded and double-stranded regions. Double-stranded nucleic acid molecules and nucleic acid molecules comprising double-stranded regions may be denatured (e.g., chemically or thermally denatured) to separate strands from one another prior to undergoing further analysis and processing. Examples of nucleic acid molecules include, but are not limited to, deoxyribonucleic acid (DNA), genomic DNA, plasmid DNA, complementary DNA (cDNA), cell-free (e.g., non-encapsulated) DNA (cfDNA), cell-free fetal DNA (cffDNA), circulating tumor DNA (ctDNA), nucleosomal DNA, chromatosomal DNA, mitochondrial DNA (miDNA), ribonucleic acid (RNA), messenger RNA (mRNA), transfer RNA (tRNA), micro RNA (miRNA), ribosomal RNA (rRNA), circulating RNA (cRNA), short hairpin RNA (shRNA), small interfering RNA (siRNA), an artificial nucleic acid analog, recombinant nucleic acid, plasmids, viral vectors, and chromatin. In some cases, RNA molecules may be reverse transcribed using a reverse transcription process to generate cDNA, which may be subjected to subsequent analysis. As shown in **FIG. 8**, the addition of RNAse H in a reverse-transcription reaction along with heating to a high temperature may ensure that remaining RNA fragments do not contributed to subsequent PCR processes.

Nucleic acid molecules may comprise one or more mutations, such as one or more somatic or germline mutations. A mutation may be naturally occurring or may be introduced using, for example, a gene editing process. Mutations may be selected from the group consisting of, but not limited to, additions, deletions, duplications, gene amplifications, gene duplications, gene truncations, base substitutions, base modification (e.g., methylation), copy number variations, gene fusions, single nucleotide polymorphisms, transversions, translocations, inversions, indels, aneuploidy, polyploidy, chromosomal fusions, chromosomal structure alterations, and DNA or chromosomal lesions.

The plurality of nucleic acid molecules of a nucleic acid sample may comprise a plurality of target nucleic acid sequences. A single nucleic acid molecule may comprise a single target sequence. Alternatively, a single nucleic acid molecule may comprise multiple target sequences. For example, a first strand of a nucleic acid molecule may comprise a first target sequence and a second strand of the nucleic acid molecule may comprise a second target sequence. Alternatively or in addition, a nucleic acid molecule of the plurality of nucleic acid molecules of a nucleic acid sample may not include a target nucleic acid sequence, or complement thereof. For example, the sample may include nucleic acid molecules including target nucleic acid sequences, or complements thereof, as well as nucleic acid molecules that do not include target nucleic acid sequences, or complements thereof. The latter nucleic acid molecules may comprise sequences complementary to one or more primers used in a directional targeted sequencing process such that off-target products may be produced.

All of a portion of the target nucleic acid sequences of the plurality of nucleic acid molecules may be known. For example, a first target sequence may be known and a second target sequence may not be known. A known target sequence may correspond to, for example, a pathogen present in or suspected or believed to be present in the nucleic acid sample. A pathogen may be, for example, a parasite, virus, fungus, or bacteria.

### Primers

Primers for use in the presently claimed methods may be designed and/or selected for use based on target sequences known, believed, or suspected of being included within a given nucleic acid sample. For example, a nucleic acid sample may be suspected of including a first target sequence corresponding to a first pathogen and a second target sequence corresponding to a second pathogen. Primers selected for use in analyzing the nucleic acid sample may include primers corresponding to both the first and second target sequences, and/or complements thereof. Primers corresponding to a plurality of different target nucleic acid sequences may be used in a given directional targeted sequencing process. For example, primers corresponding to 1, 2, 3, 4, 5, 6, 7, 8, 9, 10, 11, 12, 13, 14, 15, 16, 17, 18, 19, 20, 30, 40, 50, 60, 70, 80, 90, 100, or more target nucleic acid sequences may be used in a given directional targeted sequencing process. Accordingly, in an aspect, the present disclosure provides a kit comprising a plurality of primers configured to anneal to a plurality of nucleic acid molecules comprising a plurality of target nucleic acid sequences for use in a directional targeted sequencing process. The plurality of primers may comprise a first set of primers for use in a Multiplex PCR process and a second set of primers for use in an Asymmetric PCR process. The first set of primers may include primers configured to anneal to nucleic acid molecules in proximity to target nucleic acid sequences as well as primers configured to anneal to nucleic acid molecules in proximity to the complements of the target nucleic acid sequences. The second set of primers may be "nested" primers configured to anneal downstream of annealed first primers. All or a portion of the first set of primers may comprise one or more additional features or moieties (as described below) such as a biotin moiety and/or a portion of a sequencing adapter (e.g., a sequencing primer). All or a portion of the second set of primers may also comprise one or more additional features or moieties such as a portion of a sequencing adapter (e.g., a sequencing primer). The kit may further comprise a third set of primers for use in an FC-PCR process. The third set of primers may comprise sequencing adapters. Such sequencing adapters may comprise a sequencing primer, a flowcell attachment sequence, and an index sequence, such as a barcode sequence.

Multiplex primers may be configured to anneal to sequences adjacent to or otherwise in proximity to the target nucleic acid sequences, while nested primers utilized in Asymmetric PCR processes may be configured to anneal to a nucleic acid molecule downstream of a multiplex primer or complement thereof such, upon extension of the nested primer, the resultant extension product will include the nested primer sequence but not the multiplex primer sequence, or complement thereof.

Primers may include any useful number and sequence of bases. For example, a primer may include at least 4 bases, such as at least 5, 6, 7, 8, 9, 10, 11, 12, 13, 14, 15, 16, 17, 18, 19, 20, or more bases. A primer may include any combination of canonical and non-canonical bases. For example, a primer may include bases selected from the group consisting of thymine, uracil, guanine, adenine, and cytosine or any modified versions or analogs (naturally or non-naturally occurring) thereof. A primer may comprise one or more modified nucleotides or nucleotide analogs, such as one or more nucleotides comprising a modification in a sugar or linker moiety. Primers may be selected to target specific nucleic acid sequences. For example, a Multiplex PCR process may utilize first and second primers that are configured to anneal to regions on opposite ends of a target nucleic acid sequence, or complement thereof.

Primers may include one or more additional functional sequences or moieties. For example, a primer may include a biotin moiety that may be utilized in an avidin-biotin purification scheme. A primer may include an index or tag sequence such as a barcode sequence or unique molecule identifier. Such an index sequence may comprise at least 4 nucleotides, such as at least 4, 5, 6, 7, 8, 9, 10, 11, 12, 13, 14, 15, 16, 17, 18, 19, and 20 nucleotides. In some cases, an index sequence may comprise between 4 and 20 nucleotides. Index sequences associated with different primers may be the same or different. For example, a first set of primers may comprise a first set of index sequences that may be the same or different from one another and a second set of primers may comprise a second set of index sequences that may be the same or different from one another. Index sequences of the first set and index sequences of the second set may differ from one another. An index sequence may comprise one or more different sequences, such as a first component and a second component. In an example, all amplicons derived from a given nucleic acid sample may comprise an index sequence, the first component of which may be the same for all of the amplicons and the second component of which may vary. Amplicons from a different nucleic acid sample may comprise a different index sequence.

Primers may include sequencing adapters or portions thereof. For example, a primer used in a Multiplex PCR process may comprise a portion of a sequencing adapter, or a complement thereof. Similarly, a primer used in an Asymmetric PCR process may comprise a portion of the same or a different sequencing adapter, or a complement thereof. A sequencing adapter may include multiple components, including, but not limited to, a sequencing primer, an index sequence, and a flowcell attachment sequence. The sequencing primer may be used to direct a sequencing process such as a sequencing-by-synthesis (SBS) process. The index sequence may be used to identify a sequencing read as being associated with a given sample and/or to index sequencing reads of a collection of sequencing reads. The flowcell attachment sequence may be used to facilitate sequencing using a given sequencing platform (e.g., P5 and P7 sequences for Illumina sequencing platforms).

### Computer systems

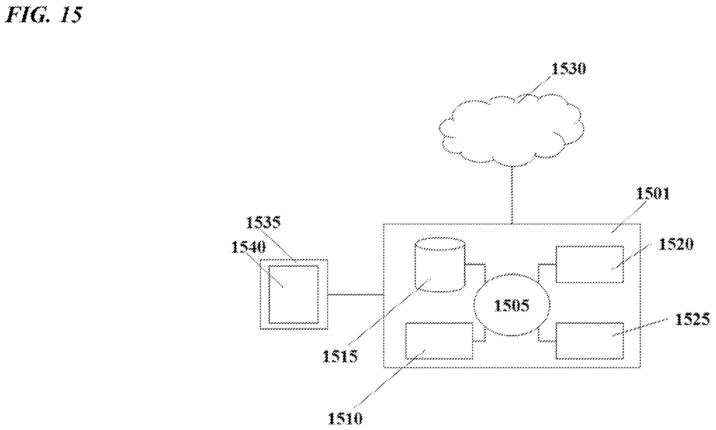

The present disclosure provides computer systems that are programmed to implement methods of the disclosure. **FIG. 15** shows a computer system **1501** that is programmed or otherwise configured to process and/or assay a sample. The computer system **1501** may regulate various aspects of sample processing and assaying of the present disclosure, such as, for example, activation of a valve or pump to transfer a reagent or sample from one chamber to another or application of heat to a sample (e.g., during an extension or amplification reaction). The computer system **1501** may be an electronic device of a user or a computer system that is remotely located with respect to the electronic device. The electronic device may be a mobile electronic device.

The computer system **1501** includes a central processing unit (CPU, also "processor" and "computer processor" herein) **1505,** which may be a single core or multi core processor, or a plurality of processors for parallel processing. The computer system **1501** also includes memory or memory location **1510** (e.g., random-access memory, read-only memory, flash memory), electronic storage unit **1515** (e.g., hard disk), communication interface **1520** (e.g., network adapter) for communicating with one or more other systems, and peripheral devices **1525,** such as cache, other memory, data storage and/or electronic display adapters. The memory **1510,** storage unit **1515,** interface **1520** and peripheral devices **1525** are in communication with the CPU **1505** through a communication bus (solid lines), such as a motherboard. The storage unit **1515** may be a data storage unit (or data repository) for storing data. The computer system **1501** may be operatively coupled to a computer network ("network") **1530** with the aid of the communication interface **1520.** The network **1530** may be the Internet, an internet and/or extranet, or an intranet and/or extranet that is in communication with the Internet. The network **1530** in some cases is a telecommunication and/or data network. The network **1530** may include one or more computer servers, which may enable distributed computing, such as cloud computing. The network **1530,** in some cases with the aid of the computer system **1501,** may implement a peer-to-peer network, which may enable devices coupled to the computer system **1501** to behave as a client or a server.

The CPU **1505** may execute a sequence of machine-readable instructions, which may be embodied in a program or software. The instructions may be stored in a memory location, such as the memory **1510.** The instructions may be directed to the CPU **1505,** which may subsequently program or otherwise configure the CPU **1505** to implement methods of the present disclosure. Examples of operations performed by the CPU **1505** may include fetch, decode, execute, and writeback.

The CPU **1505** may be part of a circuit, such as an integrated circuit. One or more other components of the system **1501** may be included in the circuit. In some cases, the circuit is an application specific integrated circuit (ASIC).

The storage unit **1515** may store files, such as drivers, libraries and saved programs. The storage unit **1515** may store user data, e.g., user preferences and user programs. The computer system **1501** in some cases may include one or more additional data storage units that are external to the computer system **1501,** such as located on a remote server that is in communication with the computer system **1501** through an intranet or the Internet.

The computer system **1501** may communicate with one or more remote computer systems through the network **1530.** For instance, the computer system **1501** may communicate with a remote computer system of a user. Examples of remote computer systems include personal computers (e.g., portable PC), slate or tablet PC's (e.g., Applet iPad, Samsung^{®} Galaxy Tab), telephones, Smart phones (e.g., Applet iPhone, Android-enabled device, Blackberry^{®}), or personal digital assistants. The user may access the computer system **1501** via the network **1530**.

Methods as described herein may be implemented by way of machine (e.g., computer processor) executable code stored on an electronic storage location of the computer system **1501,** such as, for example, on the memory **1510** or electronic storage unit **1515.** The machine executable or machine readable code may be provided in the form of software. During use, the code may be executed by the processor **1505.** In some cases, the code may be retrieved from the storage unit **1515** and stored on the memory **1510** for ready access by the processor **1505.** In some situations, the electronic storage unit **1515** may be precluded, and machine-executable instructions are stored on memory **1510.**

The code may be pre-compiled and configured for use with a machine having a processer adapted to execute the code, or may be compiled during runtime. The code may be supplied in a programming language that may be selected to enable the code to execute in a pre-compiled or as-compiled fashion.

Aspects of the systems and methods provided herein, such as the computer system **1501,** may be embodied in programming. Various aspects of the technology may be thought of as "products" or "articles of manufacture" typically in the form of machine (or processor) executable code and/or associated data that is carried on or embodied in a type of machine readable medium. Machine-executable code may be stored on an electronic storage unit, such as memory (e.g., read-only memory, random-access memory, flash memory) or a hard disk. "Storage" type media may include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system **1501** may include or be in communication with an electronic display **15155** that comprises a user interface (UI) **1540** for providing, for example, a current stage of processing or assaying of a sample (e.g., a particular operation, such as a lysis operation, that is being performed). Examples of UI's include, without limitation, a graphical user interface (GUI) and web-based user interface.

Methods and systems of the present disclosure may be implemented by way of one or more algorithms. An algorithm may be implemented by way of software upon execution by the central processing unit **1505.**

### EXAMPLES

The methods described herein may be useful for a variety of applications including, but not limited to, metagenomics, cancer diagnostics, Human variation (pharmacogenomics and ancestry), and agricultural and food analysis. The methods described herein provide flexible options for low-cost applications requiring lower-plex multiplexing. For example, the methods may utilize hybridization methods rather than next generation sequencing (NGS) analysis. In addition, the methods described herein may be useful for high complexity applications that may benefit from increased specificity and sensitivity of running serial amplification processes (e.g., serial Asymmetric Polymerase Chain Reaction [PCR] processes). The methods described herein may be used with any sequencing platform, including, but not limited to, Illumina NGS platforms, Ion Torrent (Thermo) platforms, and GeneReader (Qiagen) platforms.

### Example 1. Low Cost Directional Targeted Sequencing

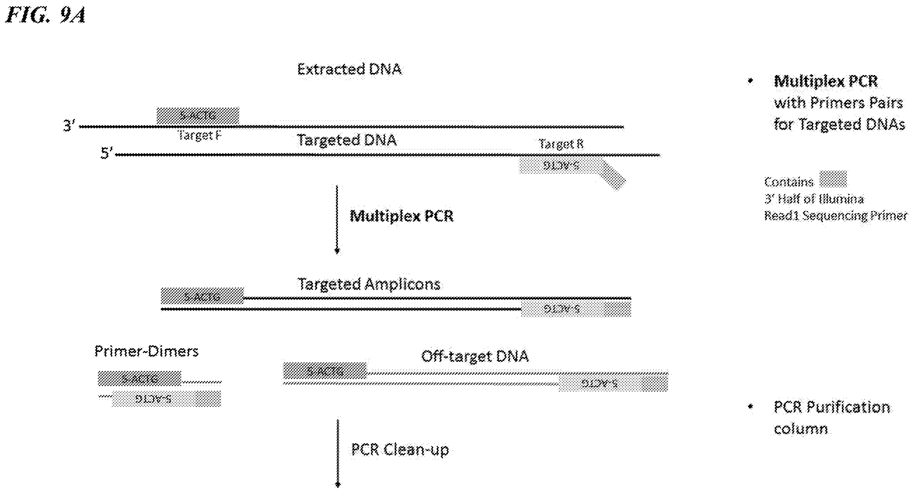

A sample comprising a plurality of nucleic acid molecules is provided. The plurality of nucleic acid molecules may comprise DNA and/or RNA. The plurality of nucleic acid molecules may comprise a plurality of target nucleic acid sequences, such as target nucleic acid sequences corresponding to one or more pathogens (e.g., viruses, bacteria, fungi, and parasites). As shown in **FIG. 9A**, a Multiplex PCR process is performed using first and second sets of primers (e.g., as described elsewhere herein). The first or second set of primers may comprise all or a portion of a first sequencing adapter (e.g., a first sequencing primer). A PCR purification column may be used to separate resultant target amplicons from other materials. In this lower cost variation, an avidin-biotin scheme is not performed. Accordingly, this method may be appropriate for applications that require a lower-plex Multiplex PCR process. With fewer primer pairs in the Multiplex PCR process, the accumulation of primer dimers may be within the binding capacity of a purification column.

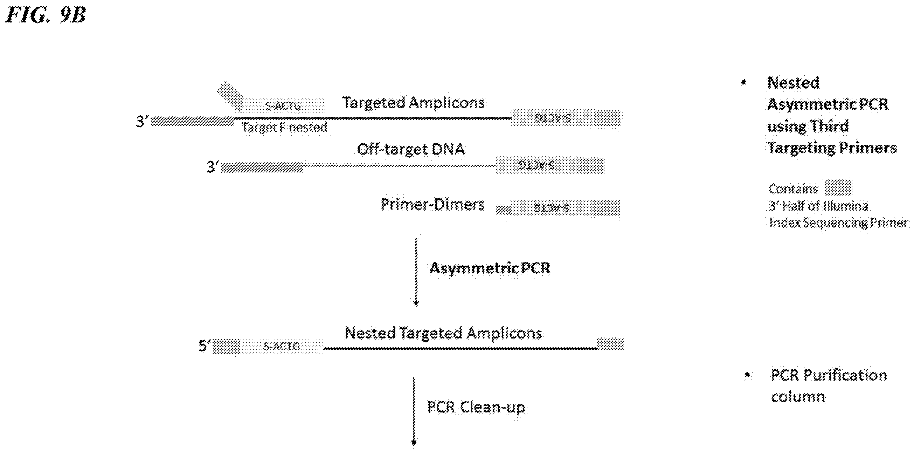

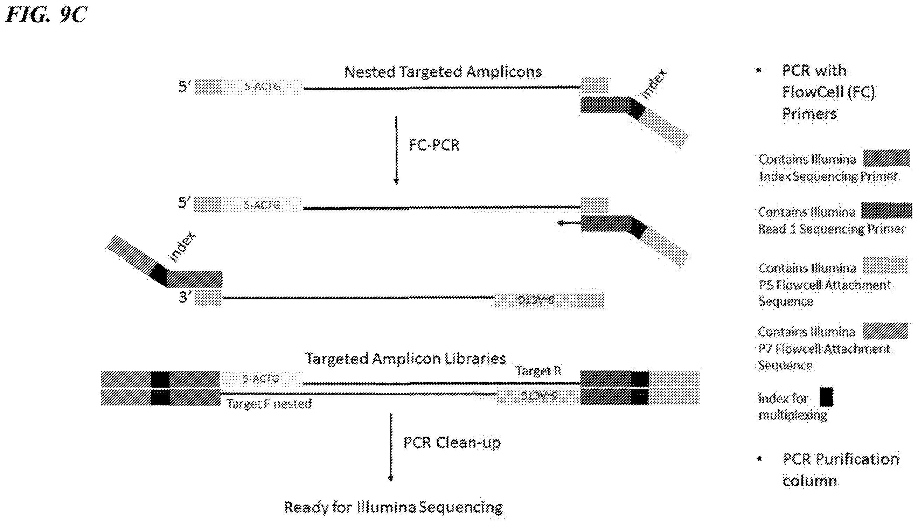

As shown in **FIG. 9B**, an Asymmetric PCR process is performed using a third targeting primer configured to anneal to a target amplicon downstream of a first or second primer, or complement thereof. The third targeting primer may comprise all or a portion of a second sequencing adapter (e.g., a second sequencing primer). The resultant nested target amplicons may comprise all or portions of both first and second sequencing adapters, or complements thereof. The nested target amplicons may be cleaned up using a second PCR purification column. As shown in **FIG. 9C****,** the nested target amplicons then undergo an FC-PCR process in which sequencing adapters are added to the nested target amplicons to prepare target amplicon libraries for sequencing. The sequencing adapters comprise sequencing primers, index sequences, and flowcell attachment sequences.

### Example 2. Directional Targeted Sequencing Using Multiple Asymmetric PCR Processes

A directional targeted sequencing method may be performed using multiple Asymmetric PCR processes. The use of serial Asymmetric PCR processes using multiple different targeting primers may facilitate increased specificity as well as increased sensitivity.

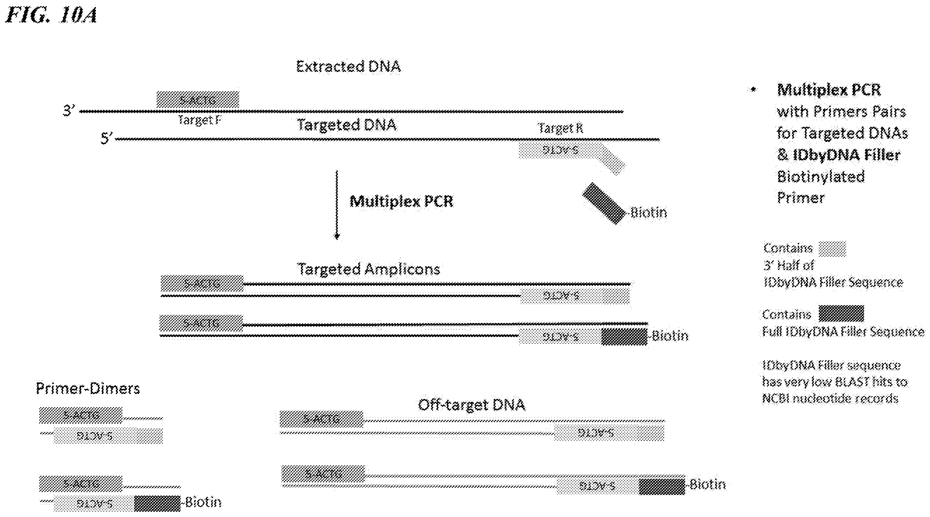

A sample comprising a plurality of nucleic acid molecules is provided. The plurality of nucleic acid molecules may comprise DNA and/or RNA. The plurality of nucleic acid molecules may comprise a plurality of target nucleic acid sequences, such as target nucleic acid sequences corresponding to one or more pathogens (e.g., viruses, bacteria, fungi, and parasites). As shown in **FIG. 10A****,** a Multiplex PCR process is performed using first and second sets of primers (e.g., as described elsewhere herein). The first or second set of primers may comprise all or a portion of a first sequencing adapter (e.g., a first sequencing primer). Some or all of the first or second set of primers comprise a filler sequence and biotin moiety, while some of none of the first or second set of primers comprise a portion of the filler sequence and no biotin moiety. The filler sequence may have low sequence identity to a natural sequence. For example, the filler sequence may have 25% or lower sequence identity to a natural sequence, such as 15% or lower, 10% or lower, or 5% or lower identity to a natural sequence. The sequence identity to a natural sequence may be determined, for example, using the Basic Local Assignment Search Tool (BLAST) to look for hits against National Center for Biotechnology Information (NCBI) records. For example, the filler sequence may have very few BLAST hits against NCBI nucleotide records. The filler sequence may be split into two halves to provide split annealing sites (e.g., as described herein). A PCR purification column may be used to separate resultant target amplicons from other materials.

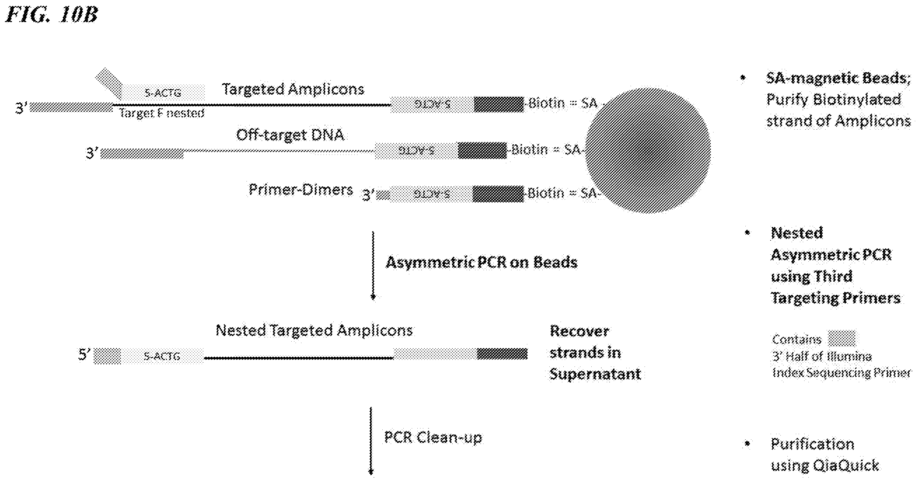

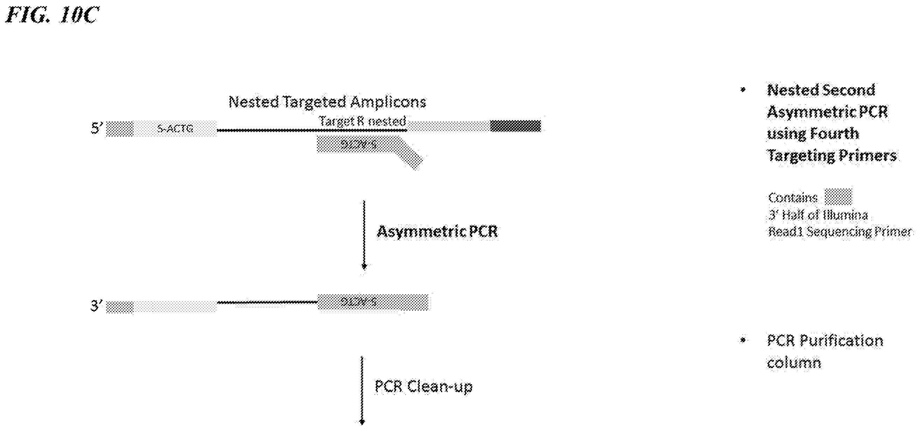

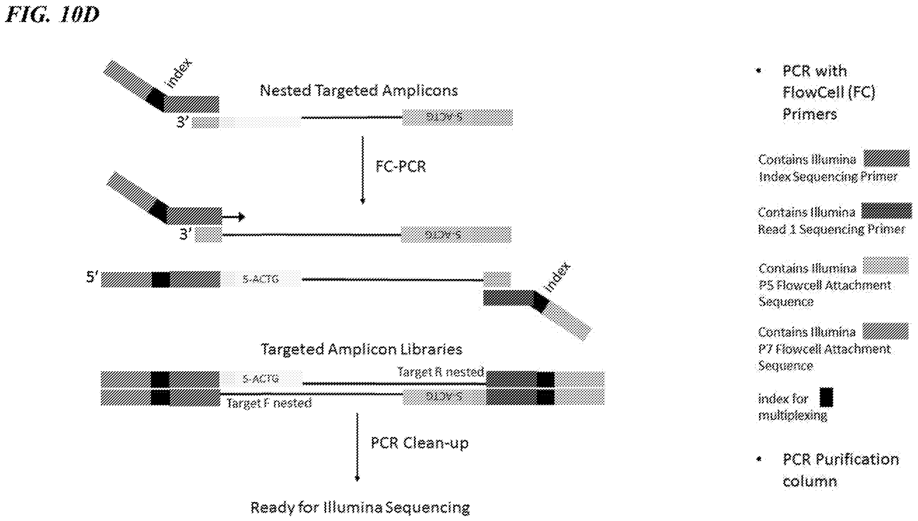

As shown in **FIG. 10B****,** an avidin-biotin scheme is used to separate materials comprising biotin moieties from those that do not comprise biotin moieties. A first Asymmetric PCR process is performed on the beads using a set of third targeting nested primers comprising all or a portion of a first sequencing adapter (e.g., a first sequencing primer) and configured to anneal to a target amplicon downstream of a first or second primer, or complement thereof, to generate a plurality of nested targeted amplicons comprising the filler sequence, or complement thereof, and the third targeting primer, or complement thereof. A purification column may be used to separate the nested targeted amplicons from other materials. As shown in **FIG. 10C****,** a second Asymmetric PCR process is performed using a set of fourth targeting nested primers comprising all of a portion of a second sequencing adapter (e.g., a second sequencing primer) and configured to anneal to a nested target amplicon downstream of a first or second primer, or complement thereof, to generate a plurality of twice-nested target amplicons. The twice-nested target amplicons may comprise the third targeting primer, or complement thereof, as well as the fourth targeting primer, or complement thereof. As shown in **FIG. 10D****,** the twice-nested target amplicons then undergo an FC-PCR process in which sequencing adapters are added to the nested target amplicons to prepare target amplicon libraries for sequencing. The sequencing adapters comprise sequencing primers, index sequences, and flowcell attachment sequences.

### Example 3. Low Cost Directional Targeted Sequencing Using Multiple Asymmetric PCR Processes

A low cost directional targeted sequencing method may be performed using multiple Asymmetric PCR processes. The use of serial Asymmetric PCR processes using multiple different targeting primers may facilitate increased specificity as well as increased sensitivity. By omitting a biotin-avidin purification scheme, the directional targeted sequencing method may be performed at lower cost than other methods described herein.

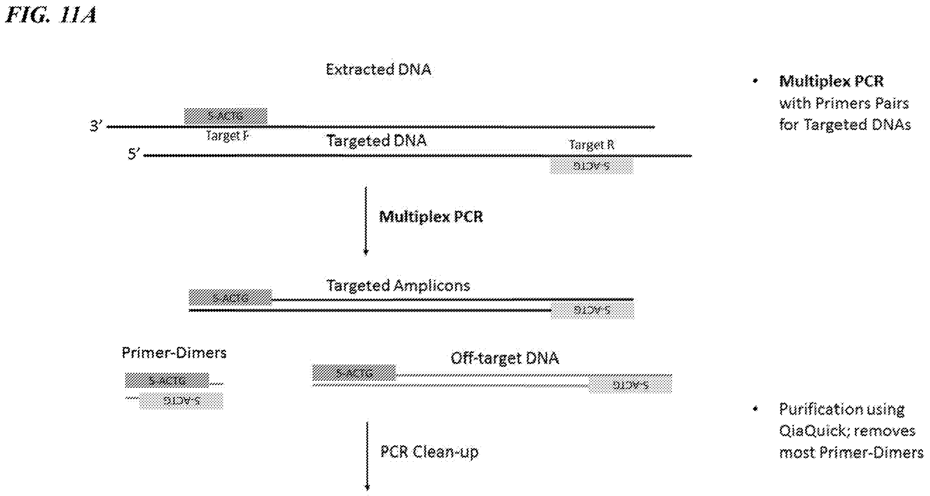

A sample comprising a plurality of nucleic acid molecules is provided. The plurality of nucleic acid molecules may comprise DNA and/or RNA. The plurality of nucleic acid molecules may comprise a plurality of target nucleic acid sequences, such as target nucleic acid sequences corresponding to one or more pathogens (e.g., viruses, bacteria, fungi, and parasites). As shown in **FIG. 11A****,** a Multiplex PCR process is performed using first and second sets of primers to generate a plurality of target amplicons comprising target nucleic acid sequences or complements thereof (e.g., as described elsewhere herein). A PCR purification column may be used to separate resultant target amplicons from other materials.

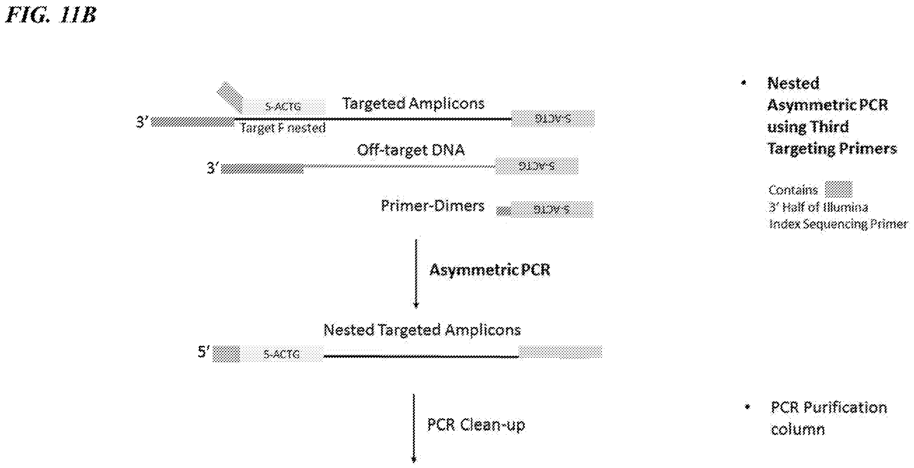

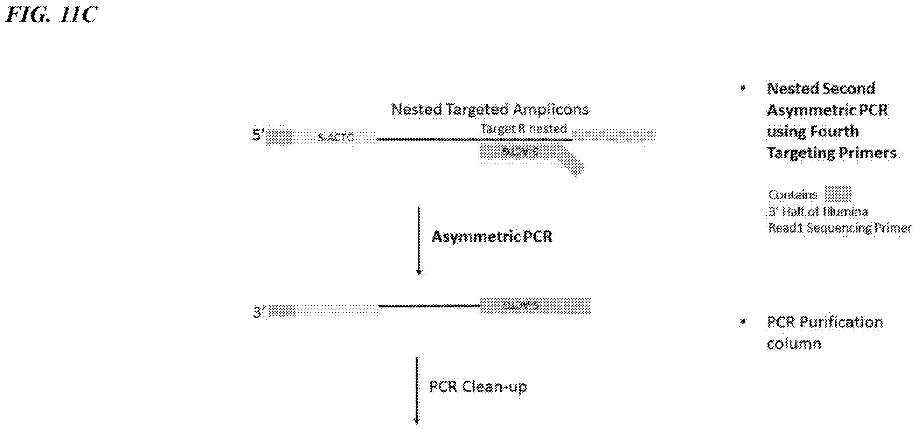

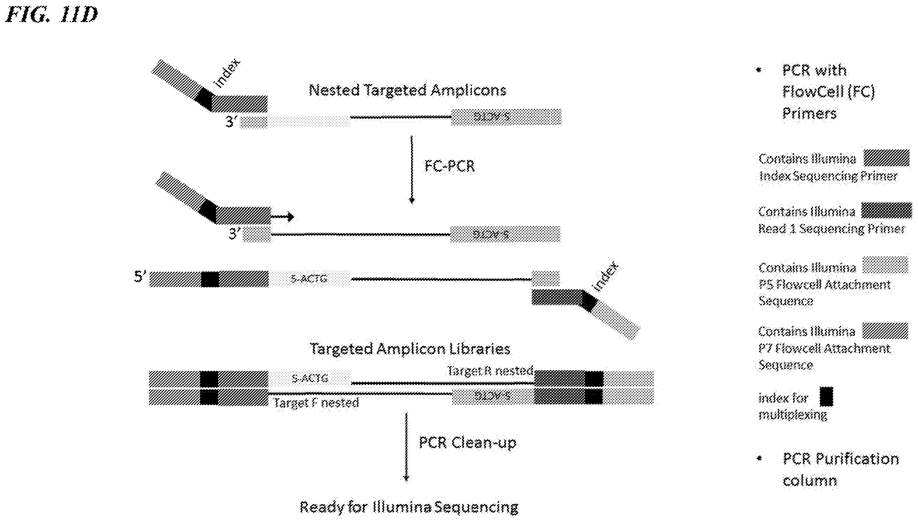

As shown in **FIG. 11B****,** a first Asymmetric PCR process is performed using a set of third targeting nested primers comprising all or a portion of a first sequencing adapter (e.g., a first sequencing primer) and configured to anneal to a target amplicon downstream of a first or second primer, or complement thereof, to generate a plurality of nested targeted amplicons comprising the the third targeting primer, or complement thereof. A purification column may be used to separate the nested targeted amplicons from other materials. As shown in **FIG. 11C****,** a second Asymmetric PCR process is performed using a set of fourth targeting nested primers comprising all of a portion of a second sequencing adapter (e.g., a second sequencing primer) and configured to anneal to a nested target amplicon downstream of a first or second primer, or complement thereof, to generate a plurality of twice-nested target amplicons. The twice-nested target amplicons may comprise the third targeting primer, or complement thereof, as well as the fourth targeting primer, or complement thereof. As shown in **FIG. 11D**, the twice-nested target amplicons then undergo an FC-PCR process in which sequencing adapters are added to the nested target amplicons to prepare target amplicon libraries for sequencing. The sequencing adapters comprise sequencing primers, index sequences, and flowcell attachment sequences.

### Example 4. Directional Targeted Sequencing Using Multiple Asymmetric PCR Processes

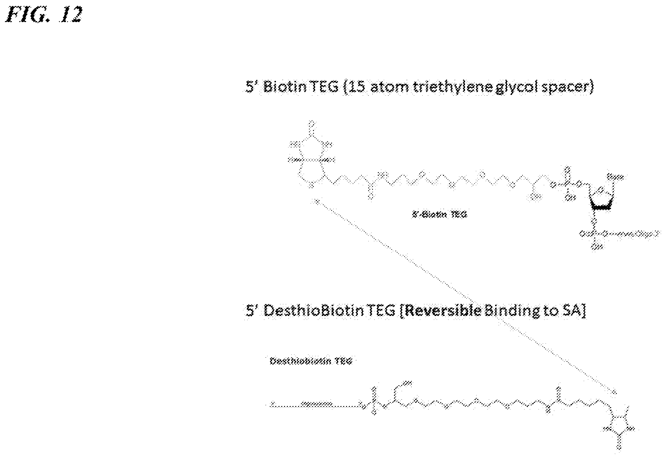

A directional targeted sequencing method may be performed using multiple Asymmetric PCR processes. The use of serial Asymmetric PCR processes using multiple different targeting primers may facilitate increased specificity as well as increased sensitivity. The Multiplex PCR process may comprise a spacer moiety designed to minimize steric hindrance from an SA-functionalized bead during the first Asymmetric PCR process. Exemplary spacers are shown in **FIG. 12** and include triethylene glycol (TEG) (e.g., 5' biotin TEG) and a 5' desthiobiotin TEG moiety. 5' biotin TEG may reduce the efficiency of an Asymmetric PCR process. Accordingly, desthiobiotin, which reversibly binds to SA-functionalized beads, may be used.

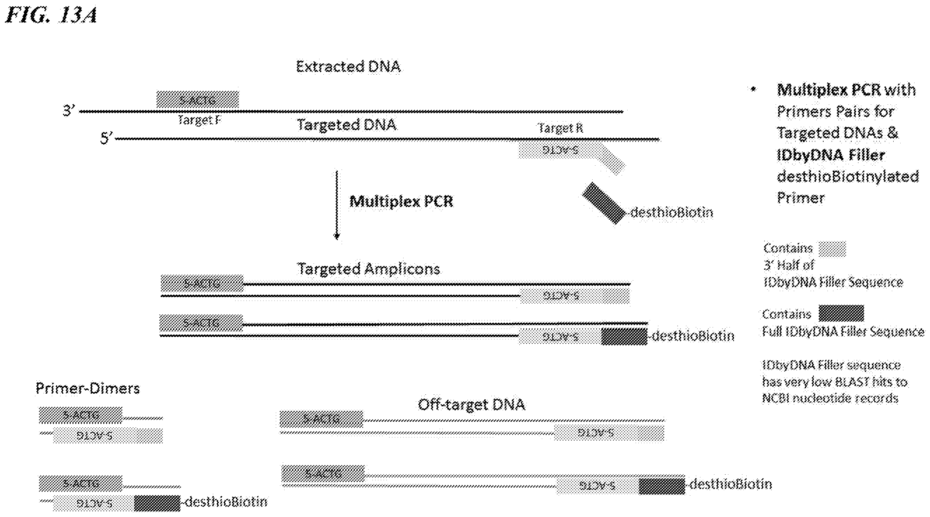

A sample comprising a plurality of nucleic acid molecules is provided. The plurality of nucleic acid molecules may comprise DNA and/or RNA. The plurality of nucleic acid molecules may comprise a plurality of target nucleic acid sequences, such as target nucleic acid sequences corresponding to one or more pathogens (e.g., viruses, bacteria, fungi, and parasites). As shown in **FIG. 13A****,** a Multiplex PCR process is performed using first and second sets of primers (e.g., as described elsewhere herein). The first or second set of primers may comprise all or a portion of a first sequencing adapter (e.g., a first sequencing primer). Some or all of the first or second set of primers comprise a filler sequence and desthiobiotin moiety, while some of none of the first or second set of primers comprise a portion of the filter sequence and no desthiobiotin moiety. The filler sequence may have low sequence identity to a natural sequence. For example, the filler sequence may have 25% or lower sequence identity to a natural sequence, such as 15% or lower, 10% or lower, or 5% or lower identity to a natural sequence. The sequence identity to a natural sequence may be determined, for example, using the Basic Local Assignment Search Tool (BLAST) to look for hits against National Center for Biotechnology Information (NCBI) records. For example, the filler sequence may have very few BLAST hits against NCBI nucleotide records. The filler sequence may be split into two halves to provide split annealing sites (e.g., as described herein). A PCR purification column may be used to separate resultant target amplicons from other materials.

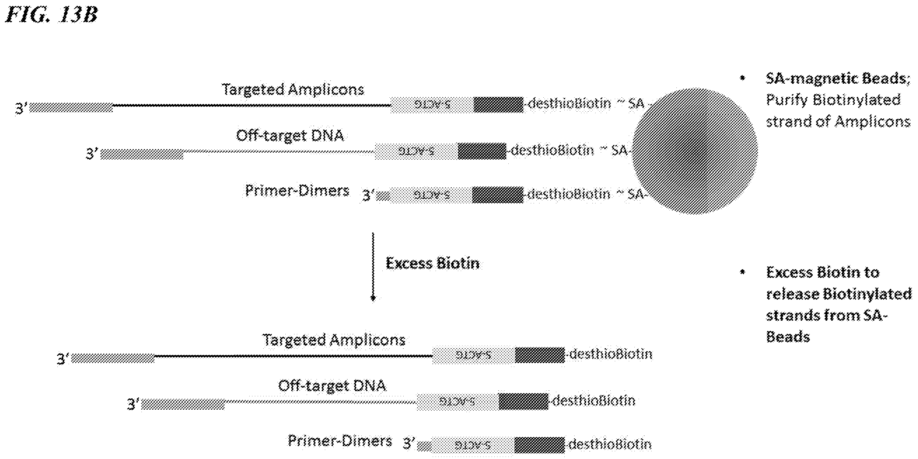

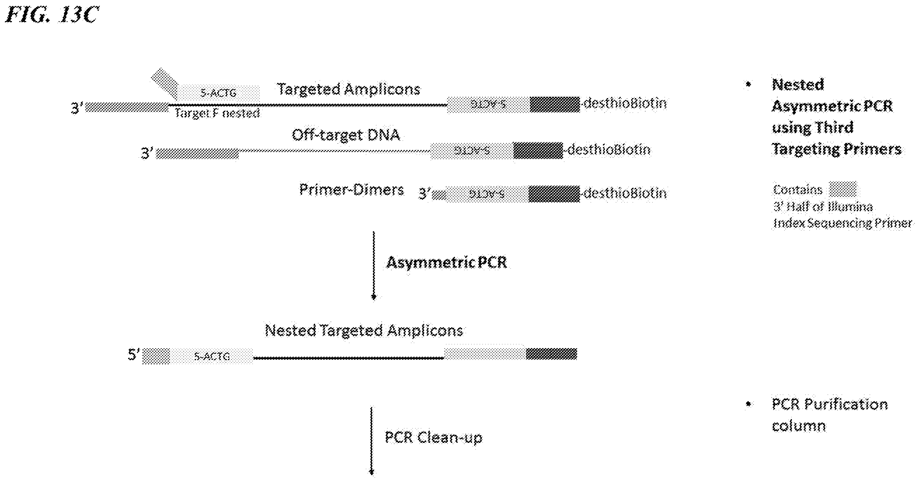

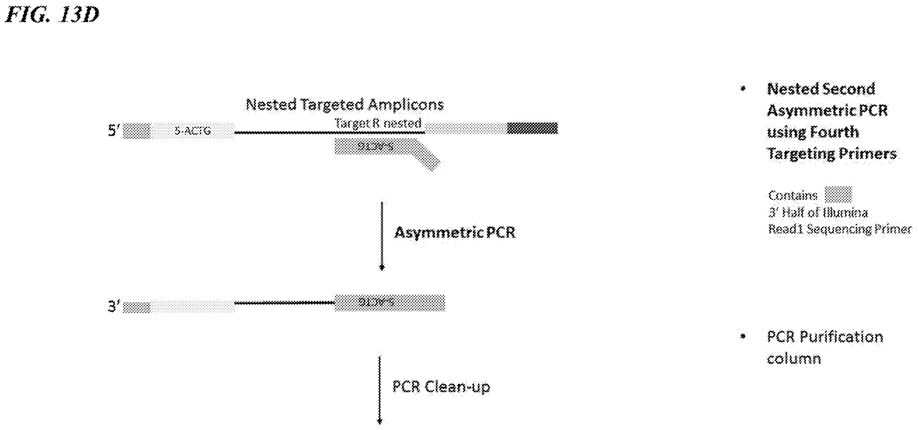

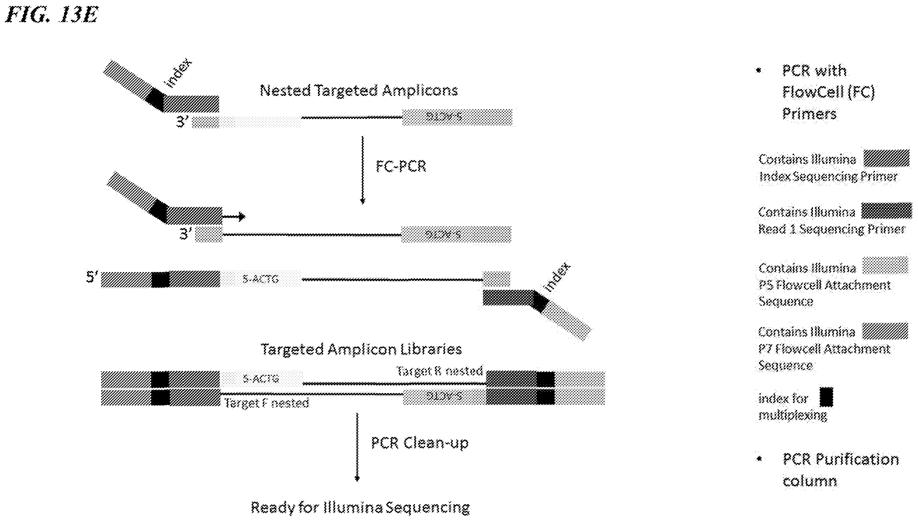

As shown in **FIG. 13B****,** an avidin-biotin scheme is used to separate materials comprising desthiobiotin moieties from those that do not comprise destihobiotin moieties. Excess biotin may then be used to remove materials comprising desthiobiotin moieties so that subsequent processes may be performed in solution. As shown in **FIG. 13C****,** a first Asymmetric PCR process is performed in solution using a set of third targeting nested primers comprising all or a portion of a first sequencing adapter (e.g., a first sequencing primer) and configured to anneal to a target amplicon downstream of a first or second primer, or complement thereof, to generate a plurality of nested targeted amplicons comprising the filler sequence, or complement thereof, and the third targeting primer, or complement thereof. A purification column may be used to separate the nested targeted amplicons from other materials. As shown in **FIG. 13D****,** a second Asymmetric PCR process is performed using a set of fourth targeting nested primers comprising all of a portion of a second sequencing adapter (e.g., a second sequencing primer) and configured to anneal to a nested target amplicon downstream of a first or second primer, or complement thereof, to generate a plurality of twice-nested target amplicons. The twice-nested target amplicons may comprise the third targeting primer, or complement thereof, as well as the fourth targeting primer, or complement thereof. As shown in **FIG. 13E****,** the twice-nested target amplicons then undergo an FC-PCR process in which sequencing adapters are added to the nested target amplicons to prepare target amplicon libraries for sequencing. The sequencing adapters comprise sequencing primers, index sequences, and flowcell attachment sequences.

### Example 5. Directional Targeted Sequencing for Hybridization-Based Applications

A directional targeted sequencing method configured for hybridization-based applications may be performed. First and second filler sequences having low BLAST hits against NCBI nucleotide records may be used in Multiplex PCR and Asymmetric PCR processes, respectively. Resultant PCR amplicons are ready for hybridization to oligonucleotide arrays or beads for read-out. Established commercial oligonucleotide hybridization methods include: GeneChip (Affymetrix Thermo), BeadArray (Illumina) and xMAP (Luminex). As such hybridization-based methods may not utilize bridge amplification, off-target primer-dimers and other non-specific products will be present in the hybridization. However, the stringency of post-hybridization washes may mitigate noise from these unwanted products.

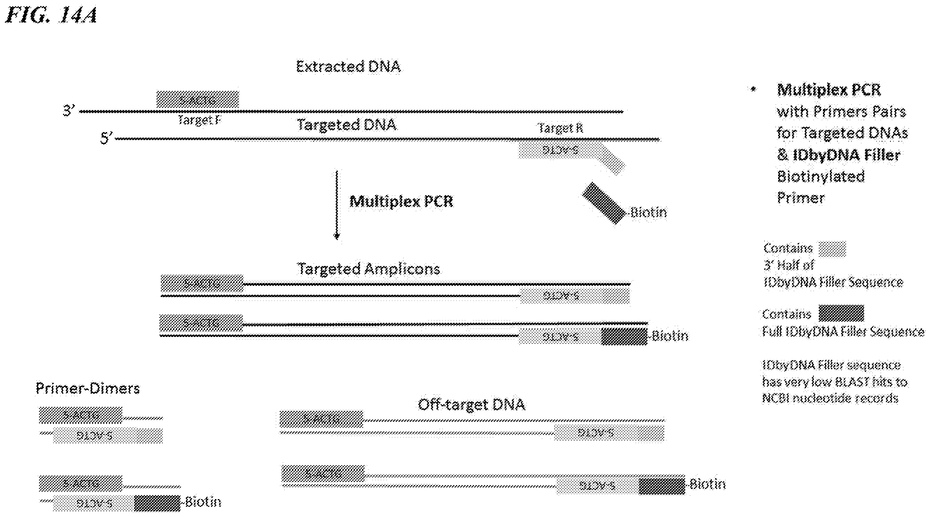

A sample comprising a plurality of nucleic acid molecules is provided. The plurality of nucleic acid molecules may comprise DNA and/or RNA. The plurality of nucleic acid molecules may comprise a plurality of target nucleic acid sequences, such as target nucleic acid sequences corresponding to one or more pathogens (e.g., viruses, bacteria, fungi, and parasites). As shown in **FIG. 14A****,** a Multiplex PCR process is performed using first and second sets of primers (e.g., as described elsewhere herein). The first or second set of primers may comprise all or a portion of a first sequencing adapter (e.g., a first sequencing primer). Some or all of the first or second set of primers comprise a first filler sequence and biotin moiety, while some of none of the first or second sets of primers comprise a portion of the filter sequence and no biotin moiety. The first filler sequence may have very few BLAST hits against NCBI nucleotide records. The first filler sequence may be split into two halves to provide split annealing sites (e.g., as described herein). A PCR purification column may be used to separate resultant target amplicons from other materials.

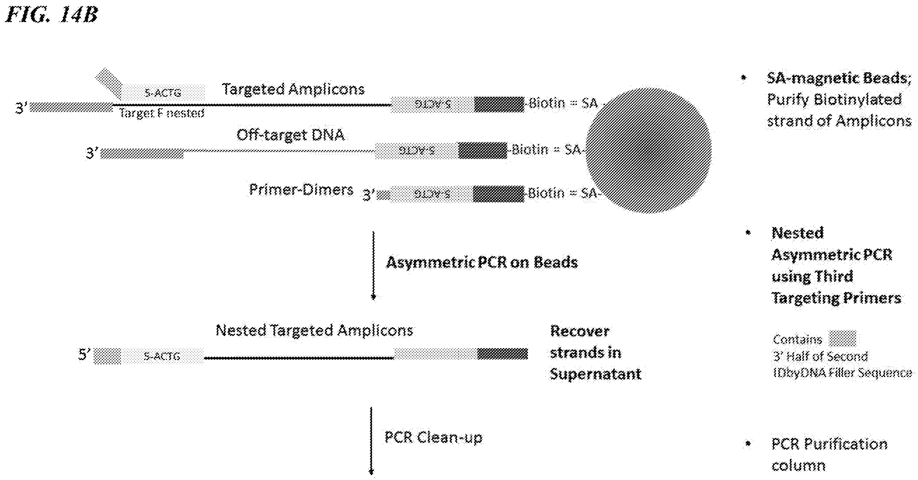

As shown in **FIG. 14B****,** an avidin-biotin scheme is used to separate materials comprising biotin moieties from those that do not comprise biotin moieties. A first Asymmetric PCR process is performed on the SA-functionalized beads using a set of third targeting nested primers comprising all or a portion of a second filler sequence, or complement thereof, and configured to anneal to a target amplicon downstream of a first or second primer, or complement thereof, to generate a plurality of nested targeted amplicons comprising the first filler sequence, or complement thereof, and the third targeting primer comprising the second filler sequence, or complement thereof. A purification column may be used to separate the nested targeted amplicons from other materials.

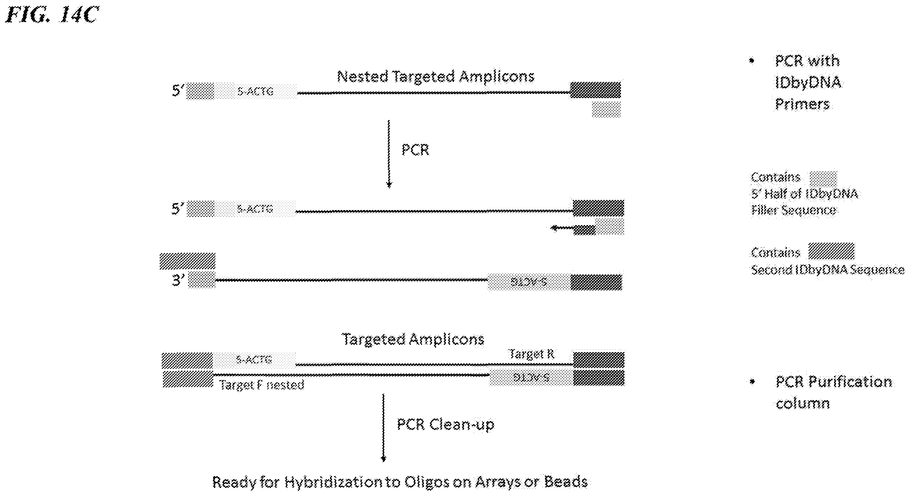

As shown in **FIG. 14C****,** the nested targeted amplicons are subjected to an additional PCR process using the second filler sequence to generate target amplicons comprising a first filler sequence, or complement thereof, at a first end and a second filler sequence, or complement thereof, at a second end. The resultant amplicons may be subjected to an additional purification process and then be provided for hybridization to oligos on arrays or beads.

Several aspects are described with reference to example applications for illustration. Unless otherwise indicated, any embodiment may be combined with any other embodiment. It should be understood that numerous specific details, relationships, and methods are set forth to provide a full understanding of the features described herein. A skilled artisan, however, will readily recognize that the features described herein may be practiced without one or more of the specific details or with other methods. The features described herein are not limited by the illustrated ordering of acts or events, as some acts may occur in different orders and/or concurrently with other acts or events. Furthermore, not all illustrated acts or events are required to implement a methodology in accordance with the features described herein.

Some inventive embodiments herein contemplate numerical ranges. When ranges are present, the ranges include the range endpoints. Additionally, every sub range and value within the range is present as if explicitly written out. The term "about" or "approximately" may mean within an acceptable error range for the particular value as determined by one of ordinary skill in the art, which will depend in part on how the value is measured or determined, e.g., the limitations of the measurement system. For example, "about" may mean within 1 or more than 1 standard deviation, per the practice in the art. Alternatively, "about" may mean a range of up to 20%, up to 10%, up to 5%, or up to 1% of a given value. Alternatively, particularly with respect to biological systems or processes, the term may mean within an order of magnitude, within 5-fold, or within 2-fold, of a value. Where particular values are described in the application and claims, unless otherwise stated the term "about" meaning within an acceptable error range for the particular value may be assumed.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

The following numbered paragraphs (paras.) contain statements of broad combinations of the inventive technical features herein disclosed:-
1. A method of analyzing a nucleic acid sample, comprising:
   (a) providing a sample comprising a plurality of nucleic acid molecules, which plurality of nucleic acid molecules comprise a target nucleic acid sequence;
   (b) subjecting said plurality of nucleic acid molecules to a condition sufficient to generate one or more copies of said target nucleic acid sequence, or complements thereof, wherein generating said one or more first copies, or complements thereof, comprises annealing first primers of a plurality of first primers and second primers of a plurality of second primers to nucleic acid molecules of said plurality of nucleic acid molecules;
   (c) separating said one or more first copies of said target nucleic acid sequence, or complements thereof, from other materials;
   (d) subjecting said one or more first copies of said target nucleic acid sequence, or complements thereof, to a condition sufficient to generate one or more second copies of said target nucleic acid sequence, or complements thereof, wherein generating said one or more second copies, or complements thereof, comprises annealing third primers of a plurality of third primers to said one or more first copies, or complements thereof;
   (e) separating said one or more second copies of said target nucleic acid sequence, or complements thereof, from other materials;
   (f) subjecting said one or more second copies of said target nucleic acid sequence, or complements thereof, to conditions sufficient to generate one or more third copies of said target nucleic acid sequence, or complements thereof, wherein generating said one or more third copies, or complements thereof, comprises annealing first sequencing adapters of a plurality of first sequencing adapters and second sequencing adapters of a plurality of second sequencing adapters to said one or more second copies, or complements thereof,

   wherein said plurality of third primers are configured to anneal to said one or more first copies, or complements thereof, downstream of said first primers or second primers, or complements thereof;
   wherein said plurality of third primers comprise a portion of a first sequencing adapter of said plurality of first sequencing adapters, or a complement thereof.
2. The method of para. 1, wherein (c) comprises using a purification column.
3. The method of para. 1 or 2, wherein (e) comprises using a purification column.
4. The method of any one of paras. 1-3, wherein first primers of said plurality of first primers and/or second primers of said plurality of second primers comprise a biotin moiety.
5. The method of para. 4, wherein first primers of said plurality of first primers comprise said biotin moiety.
6. The method of para. 5, wherein first copies, or complements thereof, of said one or more first copies, or complements thereof, comprise said biotin moiety.
7. The method of para. 6, wherein (c) comprises using streptavidin-coated beads.
8. The method of para. 7, wherein said beads are magnetic beads.
9. The method of para. 8, wherein (c) comprises bringing nucleic acid molecules into contact with said beads, thereby attaching nucleic acid molecules to said beads, and using a magnet to separate said nucleic acid molecules attached to said beads from nucleic acid molecules not attached to said beads.
10. The method of any one of paras. 1-9, wherein each first sequencing adapter of said plurality of first sequencing adapters and each second sequencing adapter of said plurality of second sequencing adapters comprise an index sequence.
11. The method of para. 10, wherein said index sequence comprises a barcode sequence.
12. The method of para. 10 or 11, wherein said index sequence comprises between 4 and 20 nucleotides.
13. The method of any one of paras. 10-12, wherein index sequences of said plurality of first sequencing adapters and said plurality of second sequencing adapters are different from one another.
14. The method of any one of paras. 1-13, wherein each first sequencing adapter of said plurality of first sequencing adapters and each second sequencing adapter of said plurality of second sequencing adapters comprise a flowcell attachment sequence.
15. The method of para. 14, wherein said flowcell attachment sequence of said plurality of first sequencing adapters is different than said flowcell attachment sequence of said plurality of second sequencing adapters.
16. The method of any one of paras. 1-15, wherein each first sequencing adapter of said plurality of first sequencing adapters comprises a sequencing primer, and wherein each third primer of said plurality of third primers comprises said sequencing primer, or a portion thereof.
17. The method of any one of paras. 1-16, wherein each first sequencing adapter of said plurality of first sequencing adapters and each second sequencing adapter of said plurality of second sequencing adapters comprise a sequencing primer.
18. The method of para. 17, wherein said sequencing primer of said plurality of first sequencing adapters is different than said sequencing primer of said plurality of second sequencing adapters.
19. The method of any one of paras. 1-18, wherein in (b), said condition comprises two or more different temperatures.
20. The method of para. 19, wherein (b) comprises thermal cycling.
21. The method of any one of paras. 1-20, wherein in (d), said condition comprises two or more different temperatures.
22. The method of para. 21, wherein (d) comprises thermal cycling.
23. The method of any one of paras. 1-22, wherein in (f), said condition comprises two or more different temperatures.
24. The method of para. 23, wherein (f) comprises thermal cycling.
25. The method of any one of paras. 1-24, wherein said sample comprises a bodily fluid.
26. The method of para. 25, wherein said bodily fluid is selected from the group consisting of blood, urine, saliva, and sweat.
27. The method of any one of paras. 1-26, wherein said plurality of nucleic acid molecules of said sample comprise a plurality of deoxyribonucleic acid (DNA) molecules.
28. The method of any one of paras. 1-27, wherein said plurality of nucleic acid molecules of said sample comprise a plurality of ribonucleic acid (RNA) molecules.
29. The method of any one of paras. 1-28, wherein said sample comprises one or more cells.
30. The method of para. 29, further comprising lysing said one or more cells of said sample.
31. The method of any one of paras. 1-30, wherein said sample derives from a patient.
32. The method of para. 31, wherein said patient has or is suspected of having a disease or disorder.
33. The method of any one of paras. 1-32, wherein said sample derives from a plurality of patients.
34. The method of para. 33, wherein said plurality of patients have or are suspected of having a disease or disorder.
35. The method of any one of paras. 1-34, wherein said target nucleic acid sequence corresponds to a pathogen.
36. The method of para. 35, wherein said pathogen is selected from the group consisting of a fungus, bacterium, virus, and parasite.
37. A method of analyzing a nucleic acid sample, comprising:
   (a) providing a sample comprising a plurality of nucleic acid molecules, which plurality of nucleic acid molecules comprise a target nucleic acid sequence;
   (b) subjecting said plurality of nucleic acid molecules to a condition sufficient to generate one or more copies of said target nucleic acid sequence, or complements thereof, wherein generating said one or more first copies, or complements thereof, comprises annealing first primers of a plurality of first primers and second primers of a plurality of second primers to nucleic acid molecules of said plurality of nucleic acid molecules;
   (c) separating said one or more first copies of said target nucleic acid sequence, or complements thereof, from other materials; and
   (d) subjecting said one or more first copies of said target nucleic acid sequence, or complements thereof, to a condition sufficient to generate one or more second copies of said target nucleic acid sequence, or complements thereof, wherein generating said one or more second copies, or complements thereof, comprises annealing third primers of a plurality of third primers to said one or more first copies, or complements thereof,

   wherein said plurality of third primers are configured to anneal to said one or more first copies, or complements thereof, downstream of said first primers or second primers, or complements thereof;
   wherein said plurality of third primers comprise a portion of a first sequencing adapter of a plurality of first sequencing adapters, or a complement thereof.
38. The method of para. 37, further comprising separating said one or more second copies of said target nucleic acid sequence, or complements thereof, from other materials.
39. The method of para. 38, wherein separating said one or more second copies of said target nucleic acid sequence, or complements thereof, comprises using a purification column.
40. The method of any one of paras. 37-39, wherein (c) comprises using a purification column.
41. The method of any one of paras. 37-40, wherein first primers of said plurality of first primers and/or second primers of said plurality of second primers comprise a biotin moiety.
42. The method of para. 41, wherein first primers of said plurality of first primers comprise said biotin moiety.
43. The method of para. 42, wherein first copies, or complements thereof, of said one or more first copies, or complements thereof, comprise said biotin moiety.
44. The method of para. 43, wherein (c) comprises using streptavidin-coated beads.
45. The method of para. 44, wherein said beads are magnetic beads.
46. The method of para. 45, wherein (c) comprises bringing nucleic acid molecules into contact with said beads, thereby attaching nucleic acid molecules to said beads, and using a magnet to separate said nucleic acid molecules attached to said beads from nucleic acid molecules not attached to said beads.
47. The method of any one of paras. 37-46, wherein each first sequencing adapter of said plurality of first sequencing adapters comprises an index sequence.
48. The method of para. 47, wherein said index sequence comprises a barcode sequence.
49. The method of para. 47 or 48, wherein said index sequence comprises between 4 and 20 nucleotides.
50. The method of any one of paras. 37-49, wherein each first sequencing adapter of said plurality of first sequencing adapters comprises a flowcell attachment sequence.
51. The method of any one of paras. 37-50, wherein each first sequencing adapter of said plurality of first sequencing adapters comprises a sequencing primer, and wherein each third primer of said plurality of third primers comprises said sequencing primer, or a portion thereof.
52. The method of any one of paras. 37-51, wherein in (b), said condition comprises two or more different temperatures.
53. The method of para. 52, wherein (b) comprises thermal cycling.
54. The method of any one of paras. 37-53, wherein in (d), said condition comprises two or more different temperatures.
55. The method of para. 54, wherein (b) comprises thermal cycling.
56. The method of any one of paras. 37-55, wherein said sample comprises a bodily fluid.
57. The method of para. 56, wherein said bodily fluid is selected from the group consisting of blood, urine, saliva, and sweat.
58. The method of any one of paras. 37-57, wherein said plurality of nucleic acid molecules of said sample comprise a plurality of deoxyribonucleic acid (DNA) molecules.
59. The method of any one of paras. 37-58, wherein said plurality of nucleic acid molecules of said sample comprise a plurality of ribonucleic acid (RNA) molecules.
60. The method of any one of paras. 37-59, wherein said sample comprises one or more cells.
61. The method of para. 60, further comprising lysing said one or more cells of said sample.
62. The method of any one of paras. 37-61, wherein said sample derives from a patient.
63. The method of para. 62, wherein said patient has or is suspected of having a disease or disorder.
64. The method of any one of paras. 37-61, wherein said sample derives from a plurality of patients.
65. The method of para. 64, wherein said plurality of patients have or are suspected of having a disease or disorder.
66. The method of any one of paras. 37-65, wherein said target nucleic acid sequence corresponds to a pathogen.
67. The method of para. 66, wherein said pathogen is selected from the group consisting of a fungus, bacterium, virus, and parasite.
68. A method of analyzing a nucleic acid sample, comprising:
   (a) providing a sample comprising a plurality of nucleic acid molecules, which plurality of nucleic acid molecules comprise a target nucleic acid sequence;
   (b) subjecting said plurality of nucleic acid molecules to a condition sufficient to generate one or more copies of said target nucleic acid sequence, or complements thereof, wherein generating said one or more first copies, or complements thereof, comprises annealing first primers of a plurality of first primers and second primers of a plurality of second primers to nucleic acid molecules of said plurality of nucleic acid molecules;
   (c) separating said one or more first copies of said target nucleic acid sequence, or complements thereof, from other materials;
   (d) subjecting said one or more first copies of said target nucleic acid sequence, or complements thereof, to conditions sufficient to generate one or more second copies of said target nucleic acid sequence, or complements thereof, wherein generating said one or more second copies, or complements thereof, comprises annealing first sequencing adapters of a plurality of first sequencing adapters and second sequencing adapters of a plurality of second sequencing adapters to said one or more first copies, or complements thereof,
   wherein said plurality of first primers comprise (i) a portion of a first sequencing adapter of said plurality of first sequencing adapters, or a complement thereof; or (ii) a filler sequence that does not have substantial sequence identity to a natural sequence.
69. The method of para. 68, wherein (c) comprises using a purification column.
70. The method of para. 68 or 69, wherein first primers of said plurality of first primers and/or second primers of said plurality of second primers comprise a biotin moiety.
71. The method of para. 70, wherein first primers of said plurality of first primers comprise said biotin moiety.
72. The method of para. 71, wherein first copies, or complements thereof, of said one or more first copies, or complements thereof, comprise said biotin moiety.
73. The method of para. 72, wherein (c) comprises using streptavidin-coated beads.
74. The method of para. 73, wherein said beads are magnetic beads.
75. The method of para. 74, wherein (c) comprises bringing nucleic acid molecules into contact with said beads, thereby attaching nucleic acid molecules to said beads, and using a magnet to separate said nucleic acid molecules attached to said beads from nucleic acid molecules not attached to said beads.
76. The method of any one of paras. 68-75, wherein each first sequencing adapter of said plurality of first sequencing adapters and each second sequencing adapter of said plurality of second sequencing adapters comprise an index sequence.
77. The method of para. 76, wherein said index sequence comprises a barcode sequence.
78. The method of para. 76 or 77, wherein said index sequence comprises between 4 and 20 nucleotides.
79. The method of any one of paras. 76-78, wherein index sequences of said plurality of first sequencing adapters and said plurality of second sequencing adapters are different from one another.
80. The method of any one of paras. 68-79, wherein each first sequencing adapter of said plurality of first sequencing adapters and each second sequencing adapter of said plurality of second sequencing adapters comprise a flowcell attachment sequence.
81. The method of para. 80, wherein said flowcell attachment sequence of said plurality of first sequencing adapters is different than said flowcell attachment sequence of said plurality of second sequencing adapters.
82. The method of any one of paras. 68-81, wherein each first sequencing adapter of said plurality of first sequencing adapters comprises a sequencing primer, and wherein each first primer of said plurality of first primers comprises said sequencing primer, or a portion thereof.
83. The method of any one of paras. 68-82, wherein each first sequencing adapter of said plurality of first sequencing adapters and each second sequencing adapter of said plurality of second sequencing adapters comprise a sequencing primer.
84. The method of para. 83, wherein said sequencing primer of said plurality of first sequencing adapters is different than said sequencing primer of said plurality of second sequencing adapters.
85. The method of any one of paras. 68-84, wherein each first primer of said plurality of first primers comprises said filler sequence.
86. The method of para. 85, wherein said filler sequence has 25% or lower sequence identity to a natural sequence.
87. The method of para. 86, wherein said filler sequence has 15% or lower sequence identity to a natural sequence.
88. The method of para. 87, wherein said filler sequence has 10% or lower sequence identity to a natural sequence.
89. The method of para. 88, wherein said filler sequence has 5% or lower sequence identity to a natural sequence.
90. The method of any one of paras. 68-89, wherein in (b), said condition comprises two or more different temperatures.
91. The method of para. 90, wherein (b) comprises thermal cycling.
92. The method of any one of paras. 68-91, wherein in (d), said condition comprises two or more different temperatures.
93. The method of para. 92, wherein (b) comprises thermal cycling.
94. The method of any one of paras. 68-93, wherein said sample comprises a bodily fluid.
95. The method of para. 94, wherein said bodily fluid is selected from the group consisting of blood, urine, saliva, and sweat.
96. The method of any one of paras. 68-95, wherein said plurality of nucleic acid molecules of said sample comprise a plurality of deoxyribonucleic acid (DNA) molecules.
97. The method of any one of paras. 68-96, wherein said plurality of nucleic acid molecules of said sample comprise a plurality of ribonucleic acid (RNA) molecules.
98. The method of any one of paras. 68-97, wherein said sample comprises one or more cells.
99. The method of para. 98, further comprising lysing said one or more cells of said sample.
100. The method of any one of paras. 68-99, wherein said sample derives from a patient.
101. The method of para. 100, wherein said patient has or is suspected of having a disease or disorder.
102. The method of any one of paras. 68-99, wherein said sample derives from a plurality of patients.
103. The method of para. 102, wherein said plurality of patients have or are suspected of having a disease or disorder.
104. The method of any one of paras. 68-103, wherein said target nucleic acid sequence corresponds to a pathogen.
105. The method of para. 104, wherein said pathogen is selected from the group consisting of a fungus, bacterium, virus, and parasite.
106. A method of analyzing a nucleic acid sample, comprising:
   (a) providing a sample comprising a plurality of nucleic acid molecules, which plurality of nucleic acid molecules comprise a target nucleic acid sequence, or a complement thereof, wherein a subset of said plurality of nucleic acid molecules further comprise a directing sequence;
   (b) subjecting said plurality of nucleic acid molecules to a condition sufficient to generate one or more first copies of said target nucleic acid sequence, or complements thereof, wherein generating said one or more first copies, or complements thereof, comprises annealing first primers of a plurality of first primers to said plurality of nucleic acid molecules;
   (c) separating said one or more first copies of said target nucleic acid sequence, or complements thereof, from other materials; and
   (d) subjecting said one or more first copies of said target nucleic acid sequence, or complements thereof, to conditions sufficient to generate one or more second copies of said target nucleic acid sequence, or complements thereof, wherein generating said one or more second copies, or complements thereof, comprises annealing first sequencing adapters of a plurality of first sequencing adapters and second sequencing adapters of a plurality of second sequencing adapters to said one or more first copies, or complements thereof,

   wherein said plurality of first primers are configured to anneal to said plurality of nucleic acid molecules downstream of said directing sequence;
   wherein said plurality of first primers comprise a portion of a first sequencing adapter of said plurality of first sequencing adapters, or a complement thereof.
107. The method of para. 106, further comprising, prior to (a), generating said plurality of nucleic acid molecules comprising said target nucleic acid sequence, or a complement thereof, and said directing sequence.
108. The method of para. 106 or 107, wherein generating said plurality of nucleic acid molecules comprises performing one or more amplification reactions.
109. The method of para. 108, wherein said one or more amplification reactions comprise one or more polymerase chain reactions.
110. The method of any one of paras. 106-109, wherein (c) comprises using a purification column.
111. The method of any one of paras. 106-110, wherein each first sequencing adapter of said plurality of first sequencing adapters and each second sequencing adapter of said plurality of second sequencing adapters comprise an index sequence.
112. The method of para. 111, wherein said index sequence comprises a barcode sequence.
113. The method of para. 111 or 112, wherein said index sequence comprises between 4 and 20 nucleotides.
114. The method of any one of paras. 111-113, wherein index sequences of said plurality of first sequencing adapters and said plurality of second sequencing adapters are different from one another.
115. The method of any one of paras. 106-114, wherein each first sequencing adapter of said plurality of first sequencing adapters and each second sequencing adapter of said plurality of second sequencing adapters comprise a flowcell attachment sequence.
116. The method of para. 115, wherein said flowcell attachment sequence of said plurality of first sequencing adapters is different than said flowcell attachment sequence of said plurality of second sequencing adapters.
117. The method of any one of paras. 106-116, wherein each first sequencing adapter of said plurality of first sequencing adapters comprises a sequencing primer, and wherein each first primer of said plurality of first primers comprises said sequencing primer, or a portion thereof.
118. The method of any one of paras. 106-117, wherein each first sequencing adapter of said plurality of first sequencing adapters and each second sequencing adapter of said plurality of second sequencing adapters comprise a sequencing primer.
119. The method of para. 118, wherein said sequencing primer of said plurality of first sequencing adapters is different than said sequencing primer of said plurality of second sequencing adapters.
120. The method of any one of paras. 106-119, wherein in (b), said condition comprises two or more different temperatures.
121. The method of para. 120, wherein (b) comprises thermal cycling.
122. The method of nay one of paras. 106-121, wherein in (d), said condition comprises two or more different temperatures.
123. The method of para. 122, wherein (b) comprises thermal cycling.
124. The method of any one of paras. 106-123, wherein said sample comprises a bodily fluid.
125. The method of para. 124, wherein said bodily fluid is selected from the group consisting of blood, urine, saliva, and sweat.
126. The method of any one of paras. 106-125, wherein said plurality of nucleic acid molecules of said sample comprise a plurality of deoxyribonucleic acid (DNA) molecules.
127. The method of any one of paras. 106-126, wherein said plurality of nucleic acid molecules of said sample comprise a plurality of ribonucleic acid (RNA) molecules.
128. The method of any one of paras. 106-127, wherein said sample comprises one or more cells.
129. The method of para. 128, further comprising lysing said one or more cells of said sample.
130. The method of any one of paras. 106-129, wherein said sample derives from a patient.
131. The method of para. 130, wherein said patient has or is suspected of having a disease or disorder.
132. The method of any one of paras. 106-129, wherein said sample derives from a plurality of patients.
133. The method of para. 132, wherein said plurality of patients have or are suspected of having a disease or disorder.
134. The method of any one of paras. 106-133, wherein said target nucleic acid sequence corresponds to a pathogen.
135. The method of para. 134, wherein said pathogen is selected from the group consisting of a fungus, bacterium, virus, and parasite.
136. A method of analyzing a nucleic acid sample, comprising:
   (a) providing a sample comprising a plurality of nucleic acid molecules, which plurality of nucleic acid molecules comprise a target nucleic acid sequence;
   (b) subjecting said plurality of nucleic acid molecules to a condition sufficient to generate one or more first copies of said target nucleic acid sequence, or complements thereof, which one or more copies, or complements thereof, comprise a first primer sequence or complement thereof at a first end and a second primer sequence or complement thereof at a second end, wherein said first primer sequence comprises a biotin moiety;
   (c) separating said one or more first copies of said plurality of target nucleic acid sequences, or complements thereof, from other materials;
   (d) subjecting said one or more first copies of said plurality of target nucleic acid sequences, or complements thereof, to a condition sufficient to generate one or more second copies of said target nucleic acid sequence, or complements thereof, which one or more second copies, or complements thereof, comprise a third primer sequence or complement thereof at said first end and said first primer sequence or complement thereof at said first end;
   (e) separating said one or more second copies of said target nucleic acid sequence, or complements thereof, from other materials;
   (f) subjecting said one or more second copies of said target nucleic acid sequence, or complements thereof, to a condition sufficient to generate one or more third copies of said target nucleic acid sequence, or complements thereof, which one or more third copies, or complements thereof, comprise a first sequencing adapter at a first end and a second sequencing adapter at a second end.
137. The method of para. 136, wherein (b) comprises annealing first primers of a plurality of first primers and second primers of a plurality of second primers to nucleic acid molecules of said plurality of nucleic acid molecules.
138. The method of para. 136 or 137, wherein (d) comprises annealing third primers of a plurality of third primers to said one or more first copies, or complements thereof.
139. The method of any one of paras. 136-138, wherein (f) comprises annealing first sequencing adapters of a plurality of first sequencing adapters and second sequencing adapters of a plurality of second sequencing adapters to said one or more second copies, or complements thereof.
140. The method of any one of paras. 136-139, wherein said plurality of third primers are configured to anneal to said one or more first copies, or complements thereof, downstream of said first primers or complements thereof.
141. The method of any one of paras. 136-140, wherein said plurality of third primers comprise a portion of a first sequencing adapter of said plurality of first sequencing adapters, or a complement thereof.
142. The method of any one of paras. 136-141, wherein (c) comprises using a purification column.
143. The method of any one of paras. 136-142, wherein (e) comprises using a purification column.
144. The method of any one of paras. 136-143, wherein (c) comprises using streptavidin-coated beads.
145. The method of para. 144, wherein said beads are magnetic beads.
146. The method of para. 145, wherein (c) comprises bringing nucleic acid molecules into contact with said beads, thereby attaching nucleic acid molecules to said beads, and using a magnet to separate said nucleic acid molecules attached to said beads from nucleic acid molecules not attached to said beads.
147. The method of any one of paras. 136-146, wherein said first sequencing adapter comprises a first index sequence and said second sequencing adapter comprises a second index sequence.
148. The method of para. 147, wherein said first and second index sequences comprise barcode sequences.
149. The method of para. 147 or 148, wherein said first and second index sequences comprise between 4 and 20 nucleotides.
150. The method of any one of paras. 147-149, wherein said first index sequence is different from said second index sequence.
151. The method of any one of paras. 136-150, wherein said first sequencing adapter comprises a first flowcell attachment sequence and said second sequencing adapter comprises a second flowcell attachment sequence.
152. The method of para. 151, wherein said first flowcell attachment sequence is different from said second flowcell attachment sequence.
153. The method of any one of paras. 136-152, wherein each first sequencing adapter of said plurality of first sequencing adapters comprises a sequencing primer, and wherein each third primer of said plurality of third primers comprises said sequencing primer, or a portion thereof.
154. The method of any one of paras. 136-153, wherein said first sequencing adapter comprises a first sequencing primer and said second sequencing adapter comprises a second sequencing primer.
155. The method of para. 154, wherein said first sequencing primer is different from said second sequencing primer.
156. The method of any one of paras. 136-155, wherein in (b), said condition comprises two or more different temperatures.
157. The method of para. 156, wherein (b) comprises thermal cycling.
158. The method of any one of paras. 136-157, wherein in (d), said condition comprises two or more different temperatures.
159. The method of para. 158, wherein (b) comprises thermal cycling.
160. The method of any one of paras. 136-159, wherein in (f), said condition comprises two or more different temperatures.
161. The method of para. 160, wherein (f) comprises thermal cycling.
162. The method of any one of paras. 136-161, wherein said sample comprises a bodily fluid.
163. The method of para. 162, wherein said bodily fluid is selected from the group consisting of blood, urine, saliva, and sweat.
164. The method of any one of paras. 136-163, wherein said plurality of nucleic acid molecules of said sample comprise a plurality of deoxyribonucleic acid (DNA) molecules.
165. The method of any one of paras. 136-164, wherein said plurality of nucleic acid molecules of said sample comprise a plurality of ribonucleic acid (RNA) molecules.
166. The method of any one of paras. 136-165, wherein said sample comprises one or more cells.
167. The method of para. 166, further comprising lysing said one or more cells of said sample.
168. The method of any one of paras. 136-167, wherein said sample derives from a patient.
169. The method of para. 168, wherein said patient has or is suspected of having a disease or disorder.
170. The method of any one of paras. 136-167, wherein said sample derives from a plurality of patients.
171. The method of para. 170, wherein said plurality of patients have or are suspected of having a disease or disorder.
172. The method of any one of paras. 136-171, wherein said target nucleic acid sequence corresponds to a pathogen.
173. The method of para. 172, wherein said pathogen is selected from the group consisting of a fungus, bacterium, virus, and parasite.
174. A kit comprising:
   (a) a plurality of first primers, which plurality of first primers are configured to anneal to nucleic acid molecules comprising a target nucleic acid sequence;
   (b) a plurality of second primers, which plurality of second primers are configured to anneal to nucleic acid molecules comprising complements of said target nucleic acid sequence; and
   (c) a plurality of third primers, which plurality of third primers are configured to anneal to nucleic acid molecules adjacent to said target nucleic acid sequence or complements thereof.
175. The kit of para. 174, wherein all or a portion of said plurality of first primers or said plurality of second primers comprise a biotin moiety.
176. The kit of para. 175, wherein all or a portion of said plurality of first primers comprise said biotin moiety.
177. The kit of para. 176, wherein said biotin moiety is a desthiobiotin moiety.
178. The kit of any one of paras. 175-177, further comprising a plurality of streptavidin-functionalized magnetic beads.
179. The kit of any one of paras. 174-178, wherein said plurality of first primers or said plurality of second primers comprise all or a portion of a sequencing adapter.
180. The kit of para. 179, wherein said plurality of first primers or said plurality of second primers comprise all or a portion of a sequencing primer.
181. The kit of para. 180, wherein said plurality of first primers comprise said sequencing primer.
182. The kit of any one of paras. 174-181, further comprising a plurality of first sequencing adapters and a plurality of second sequencing adapters.
183. The kit of para. 182, wherein each first sequencing adapter of said plurality of first sequencing adapters comprises a first flowcell attachment sequence.
184. The kit of para. 182 or 183, wherein each first sequencing adapter of said plurality of first sequencing adapters comprises a first sequencing primer.
185. The kit of any one of paras. 182-184, wherein each first sequencing adapter of said plurality of first sequencing adapters comprises a first index sequence.
186. The kit of any one of paras. 182-185, wherein each second sequencing adapter of said plurality of second sequencing adapters comprises a second flowcell attachment sequence.
187. The kit of any one of paras. 182-186, wherein each second sequencing adapter of said plurality of second sequencing adapters comprises a second sequencing primer.
188. The kit of any one of paras. 182-187, wherein each second sequencing adapter of said plurality of second sequencing adapters comprises a second index sequence.
189. The kit of any one of paras. 174-188, wherein said target nucleic acid sequence corresponds to a pathogen.
190. The kit of para. 189, wherein said pathogen is selected from the group consisting of a parasite, virus, bacterium, and fungus.
191. The kit of any one of paras. 174-190, further comprising one or more reagents selected from the group consisting of polymerases, buffers, solvents, and deoxyribonucleotides molecules (dNTPs).

## Claims

1. A method of analyzing a nucleic acid sample, comprising:
(a) providing a sample comprising a plurality of nucleic acid molecules, wherein the plurality of nucleic acid molecules comprises a target nucleic acid sequence;
(b) generating one or more first copies of the target nucleic acid sequence, or a complement thereof, by annealing first primers and second primers to the plurality of nucleic acid molecules;
(c) separating the one or more first copies of the target nucleic acid sequence, or complements thereof, from other materials;
(d) generating one or more second copies of the target nucleic acid sequence, or complements thereof, by annealing first sequencing adapters and second sequencing adapters to the one or more first copies, or complements thereof,
wherein the first primers comprise a filler sequence that does not have substantial sequence identity to a natural sequence.

2. The method of claim 1, wherein separating the one or more first copies comprises separating the one or more first copies using a purification column.

3. The method of claim 1 or 2, wherein the first primers and/or the second primers comprise a biotin moiety.

4. The method of claim 3, wherein the one or more first copies comprises the biotin moiety, optionally wherein the separating of the one or more first copies further comprises using beads.

5. The method of claim 4, wherein the beads are magnetic, and
wherein the separating of the one or more first copies further comprises:
contacting the nucleic acid molecules with the beads, thereby attaching nucleic acid molecules to the beads, and
using a magnet to separate the nucleic acid molecules attached to the beads from nucleic acid molecules not attached to the beads.

6. The method of any one of claims 1-5, wherein each of the first sequencing adapters and each of the second sequencing adapters comprises either:
(i) an index sequence, optionally wherein said index sequence comprises a barcode sequence, and further optionally wherein said index sequence comprises between 4 and 20 nucleotides, or
(ii) a flowcell attachment sequence, optionally wherein the flowcell attachment sequence of the first sequencing adapters is different than the flowcell attachment sequence of the second sequencing adapters.

7. The method of claim 6, wherein index sequences of the first sequencing adapters and the second sequencing adapters are different from one another.

8. The method of any one of claims 1-7, wherein each of the first sequencing adapters comprises a sequencing primer,
wherein each of the first primers comprises the sequencing primer, or a portion thereof,
optionally wherein each of the second sequencing adapters comprises a sequencing primer, and
optionally wherein the sequencing primer of the first sequencing adapters is different than the sequencing primer of the second sequencing adapters.

9. The method of any one of claims 1-8, wherein the filler sequence has 25% or lower sequence identity to the natural sequence.

10. The method of any one of claims 1-9, wherein the generating or the separating of the one or more first copies comprises thermal cycling at two or more different temperatures.

11. The method of any one of claims 1-10, wherein the sample comprises a bodily fluid, optionally wherein the bodily fluid is selected from the group consisting of blood, urine, saliva, and sweat.

12. The method of any one of claim 1-11, wherein the plurality of nucleic acid molecules comprises a plurality of deoxyribonucleic acid (DNA) molecules and/or a plurality of ribonucleic acid (RNA) molecules.

13. The method of any one of claims 1-12, wherein the sample comprises one or more cells, and the method further comprises lysing the one or more cells.

14. The method of any one of claims 1-13, wherein the sample derives from a patient or a plurality of patients that is suspected of having a disease or disorder.

15. The method of any one of claims 1-14, wherein the target nucleic acid sequence corresponds to a pathogen, and wherein the pathogen is selected from the group consisting of a fungus, bacterium, virus, and parasite.
